# EUROPÄISCHE PATENTSCHRIFT

(11) **EP 1 248 768 B1**
(45) Veröffentlichungstag und Bekanntmachung des Hinweises auf die Patenterteilung: **16.11.2005**
(21) Anmeldenummer: 00958461.6
(22) Anmeldetag: 17.08.2000
(51) Int. Cl.: C07C 279/24, C07J 9/00, C07J 41/00, A61K 31/575, A61P 1/16

(54) **GALLENSÄURE-SUBSTITUIERTE PHENYL-ALKENOYLGUANIDINE, VERFAHREN ZU IHRER HERSTELLUNG, IHRE VERWENDUNG ALS MEDIKAMENTE ODER DIAGNOSTIKA SOWIE SIE ENTHALTENDE MEDIKAMENTE**
BILE ACID-SUBSTITUTED PHENYL-ALKENOYLGUANIDINES, METHOD FOR PRODUCING SAID PHENYL-ALKENOYLGUANIDINES, USE THEREOF AS MEDICAMENTS OR DIAGNOSTIC REAGENTS AND MEDICAMENTS CONTAINING THE SAME
PHENYL-ALCENOYLGUANIDINE SUBSTITUEE PAR L'ACIDE BILIAIRE, SON PROCEDE DE PRODUCTION, SON UTILISATION COMME MEDICAMENT OU PRODUIT DE DIAGNOSTIC ET MEDICAMENTS LES CONTENANT

(30) Priorität: 02.09.1999 DE 19941764
(43) Veröffentlichungstag der Anmeldung: 16.10.2002
(73) Patentinhaber: Sanofi-Aventis Deutschland GmbH, 65929 Frankfurt am Main (DE)
(72) Erfinder: KLEEMANN, Heinz-Werner, 65474 Bischofsheim (DE); HOFMEISTER, Armin, 55283 Nierstein (DE); FALK, Eugen, 60529 Frankfurt (DE); JANSEN, Hans-Willi, 65527 Niedernhausen (DE); SCHAEFER, Hans-Ludwig, 65239 Hochheim (DE)
(86) Internationale Anmeldenummer: PCT/EP2000/008025
(87) Internationale Veröffentlichungsnummer: WO 2001/017954

(56) Entgegenhaltungen:
- EP-A- 0 624 594
- WO-A-00/24761

## Beschreibung

Die Erfindung betrifft substituierte Acylguanidine und deren pharmazeutisch verträgliche Salze und physiologisch funktionelle Derivate.

EP 0 624 594 offenbart monomere Gallensäurederivate, die sich als Hypolipidämika eignen.

Die Bildung von Gallensteinen wird neben einer Reihe von Faktoren wesentlich durch die Zusammensetzung der Galle bestimmt, im besonderen durch die Konzentration und das Verhältnis von Cholesterin, Phospholipiden und Gallensalzen. Voraussetzung für die Bildung von Cholesteringallesteinen ist das Vorhandensein einer an Cholesterin übersattigten Galle (Lit. Carey, M. C. and Small, D.M. (1978) The physical chemistry of cholesterol solubility in bile. Relationship to gallstone formation and dissolution in man, J. Clin. Invest. 61: 998-1026).

Gallensteine werden bislang vorwiegend chirurgisch entfernt, so daß ein großer therapeutischer Bedarf zur medikamentösen Gallensteinauflösung und zur Prävention der Gallensteinbildung besteht.

Der Erfindung lag die Aufgabe zugrunde, Verbindungen zur Verfügung zu stellen, die in der Lage sind, die Bildung von Gallensteinen zu verhindern, indem sie die Übersättigung der Galle mit Cholesterin verhindern, oder indem sie die Bildung von Cholesterinkristallen aus übersättigten Gallen verzögern.

Die Erfindung betrifft daher Verbindungen der Formel I 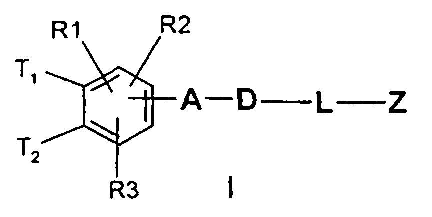 worin bedeuten:
- T1 und T2: unabhängig voneinander 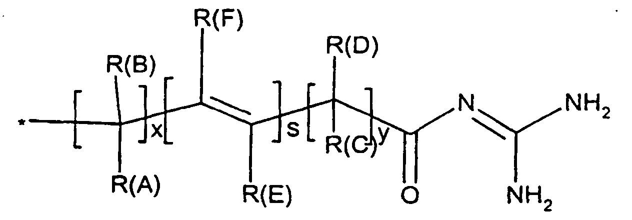 oder Wasserstoff, wobei
T1 und T2 nicht gleichzeitig Wasserstoff sein können;
- Z: 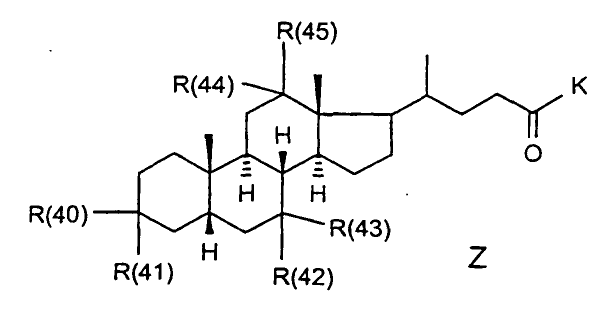
- A: eine Bindung, (C₁-C₄)-alkyl, (C₀-C₄)-alkyl-X;
- X: -O-, -CO-, -CH[OH]-, -CH[OCH₃]-, -SO₍₀₋₂₎- oder -NH-, -N(CH₃)-;
- D: Phenylen, welches nicht substituiert oder substituiert ist mit 1 - 3 Substituenten ausgewählt aus der Gruppe bestehend aus F, Cl, -CF₃, (C₁-C₄)-Alkyl, Hydroxy, Methoxy, -NH₂, NHCH₃, N(CH₃)₂ , CH₃SO₂- und H₂NO₂S-;
- R(A), R(B), R(C), R(D), R(E), R(F): unabhängig voneinander Wasserstoff, F, Cl, Br, I, CN, OH, OR(6), NR(7)R(8), (C₁-C₈)-Alkyl, O-(C₁-C₁₂)-Alkyl, (C₃-C₈)-Cycloalkyl, wobei in den Alkylresten ein, mehrere oder alle Wasserstoffe durch Fluor ersetzt sein können;
- R(6): (C₃-C₆)-Alkenyl, (C₃-C₈)-Cycloalkyl, Phenyl oder Benzyl, wobei der Phenylkern bis zu dreifach substituiert sein kann mit F, Cl, CF₃, Methyl, Methoxy, NR(9)R(10);
- R(9), R(10): unabhängig voneinander H, (C₁-C₄)-Alkyl oder (C₁-C₄)-Perfluoralkyl;
- R(7) und R(8): unabhängig voneinander (C₁-C₄)-Alkyl, (C₃-C₆)-Alkenyl, (C₃-C₈)-Cycloalkyl, Phenyl oder Benzyl, wobei der Phenylkern bis zu dreifach substituiert sein kann mit F, Cl, CF₃, Methyl, Methoxy, NR(9)R(10); oder
- R(7) und R(8): bilden gemeinsam eine Kette von 4 oder 5 Methylengruppen, von denen eine CH₂-Gruppe durch Sauerstoff, Schwefel, NH, N-CH₃ oder N-Benzyl ersetzt sein kann;
- s: Null oder 1,
- x: Null, 1 oder 2;
- y: Null, 1 oder 2;
- R(1), R(2), R(3): unabhängig voneinander Wasserstoff, F, Cl, Br, I, CN, -(C=O)- N=C(NH₂)₂, -SO_{(0-1 )}-(C₁-C₈)-Alkyl , O-(C₀-C₄)-Alkyl-Phenyl, -(C₀-C₄)-Alkyl-Phenyl, wobei der Phenylkern bis zu dreifach substituiert sein kann mit F, Cl, CF₃, Methyl, Methoxy, -(C₀-C₄)-Alkyl-NR(21)R(22); (C₁-C₈)-Alkyl, O-(C₁-C₁₂)-Alkyl, (C₃-C₈)-Cycloalkyl, wobei in den Alkylresten ein, mehrere oder alle Wasserstoffe durch Fluor ersetzt sein können;
- R(21), R(22): unabhängig voneinander H, (C₁-C₄)-Alkyl;
- L: (C₁-C₁₅)-Alkyl , wobei eine oder mehrere (CH₂)-Gruppen ersetzt sein können durch -CH=CH-, -C≡C-, -O-, -NR(47)-, -NR(48)-, -CO-, -SO₂-;
- R(47): Wasserstoff, (C₁-C₈)-Alkyl, R(48)-CO-, Phenyl, Benzyl;
- R(48): Wasserstoff, (C₁-C₈)-Alkyl, Phenyl, (CH₂)-Phenyl, wobei der Phenylkern bis zu dreifach substituiert sein kann mit F, Cl, CF₃, Methyl, Methoxy;
- R(40) bis R(45): unabhängig voneinander H, -O R(50), -S R(50), NH R(50), -N R(50)₂, -O-(CO)- R(50), -S-(CO)- R(50), -NH-(CO)- R(50), -O-PO-(O R(50))-O R(50), -O-(SO₂)-O R(50), -R(50), eine Bindung zu L;oder
- R(40) und R(41), R(42) und R(43), R(44) und R(45): jeweils gemeinsam der Sauerstoff einer Carbonylgruppe;
wobei immer genau einer der Reste die Bedeutung einer Bindung zu L hat;
- R(50): Wasserstoff, (C₁-C₄)-Alkyl, Phenyl, (CH₂)-Phenyl, wobei der Phenylkern bis zu dreifach substituiert sein kann mit F, Cl, CF₃, Methyl, Methoxy;
- K: -OR(51), -NH(R51), -N(R51)₂, -HN-CH₂-CH₂-CO₂H, -HN-CH₂-CH₂-SO₃H, -N(CH₃)CH₂CO₂H, -HN-CH(R46)CO₂H, -OKa, wobei Ka ein Kation bedeutet, wie z.B. ein Alkali- oder Erdalkaliion oder ein quartäres Ammoniumion;
- R(46): Wasserstoff, C₁-C₄-Alkyl, Benzyl, -CH₂-OH, H₃CSCH₂CH₂-, HO₂CCH₂-, HO₂CCH₂CH₂-;
- R(51): H, (C₁-C₄)-Alkyl, Phenyl, (CH2)-Phenyl, wobei der Phenylrest bis zu dreifach substituiert sein kann mit F, Cl, CF₃, Methyl, Methoxy;
sowie deren pharmazeutisch verträgliche Salze und physiologisch funktionelle Derivate.

Bevorzugt sind Verbindungen der Formel I, 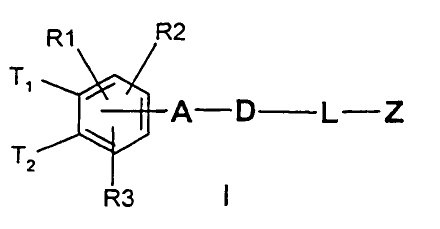 worin ein oder mehrere Rest(e) die folgende Bedeutung hat bzw. haben:
- T1 und T2: unabhängig voneinander gleich 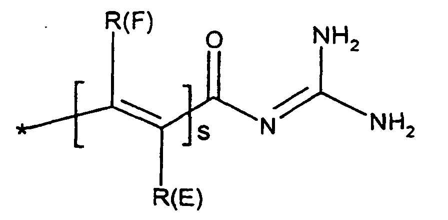 oder Wasserstoff, wobei
T1 und T2 nicht gleichzeitig Wasserstoff sein können,
- L-Z: 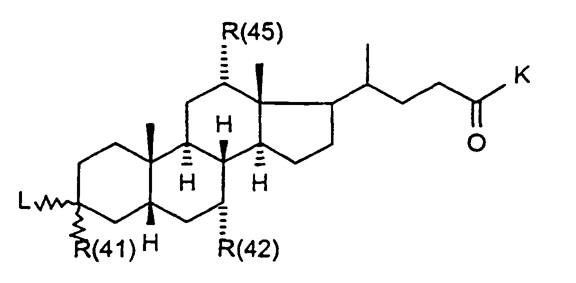
- A: eine Bindung -CH₂-, CH₂-X-;
- X: -O-, -CO-, -CH[OH]-, -CH[OCH₃]-, -SO₍₀₋₂₎- oder -NH-, N(CH₃)-;
- s: Null oder 1;
- D: Phenylen, welches bis zu zweifach substituiert sein kann mit , F, Cl, -CF₃, (C₁-C₄)-Alkyl, Hydroxy, Methoxy, -NH₂, NHCH₃, N(CH₃)₂, CH₃SO₂-, H₂NO₂S-;
- R(E): F, Cl, CN, OR(12), (C₁-C₄)-Alkyl, O-(C₁-C₄)-Alkyl, (C₃-C₆)-Cycloalkyl, wobei in den Alkylresten ein, mehrere oder alle Wasserstoffe durch Fluor ersetzt sein können;
- R(6): (C₃-C₆)-Alkenyl, (C₃-C₈)-Cycloalkyl, Phenyl oder Benzyl, wobei der Phenylkern bis zu dreifach substituiert sein kann mit F, Cl, CF₃, Methyl, Methoxy, NR(9)R(10);
- R(9), R(10): unabhängig voneinander H, (C₁-C₄)-Alkyl oder (C₁-C₄)-Perfluoralkyl;
- R(F): Wasserstoff;
- R(1), R(2), R(3): unabhängig voneinander Wasserstoff, F, Cl, Br, I, CN, -(C=O)- N=C(NH₂)₂, -SO₍₀₋₁₎-(C₁-C₈)-Alkyl , O-(C₀-C₄)-Alkyl-Phenyl, -(C₀-C₄)-Alkyl-Phenyl, wobei der Phenylkern bis zu dreifach substituiert sein kann mit F, Cl, CF₃, Methyl, Methoxy;
- L: (C₁-C₈)-Alkyl , wobei eine oder mehrere (CH₂)-Gruppen ersetzt sein können durch -CH=CH-, -C≡C-, -O-, -NR(47)-, -NR(48)-, -CO-, -SO₂-;
- R(47): Wasserstoff, (C₁-C₄)-Alkyl, R(48)-CO-, Phenyl, (CH₂)-Phenyl;
- R(48): Wasserstoff, (C₁-C₄)-Alkyl, Phenyl, (CH₂)-Phenyl, wobei der Phenylkern bis zu dreifach substituiert sein kann mit F, Cl, CF₃, Methyl, Methoxy;
- R(41), R(42), R(45): unabhängig voneinander H, -O R(50), -S R(50), NH R(50), -N R(50)₂, -O-(CO)- R(50), -S-(CO)- R(50), -NH-(CO)- R(50), -O-PO-(O R(50))-O R(50), -O-(SO₂)-O R(50), - R(50);
- R(50): Wasserstoff, (C₁-C₄)-Alkyl, Phenyl, (CH₂)-Phenyl, wobei der Phenylkern bis zu dreifach substituiert sein kann mit F, Cl, CF₃, Methyl, Methoxy;
- K: -OR(51), -NH(R51), -N(R51)₂, -HN-CH₂-CH₂-CO₂H, -HN-CH₂-CH₂-SO₃H, -N(CH₃)CH₂CO₂H, -HN-CH(R46)CO₂H, -OKa, wobei Ka ein Kation bedeutet, wie z.B. ein Alkali- oder Erdalkaliion oder ein quartäres Ammoniumion,
- R(46): H, C₁-C₄-Alkyl, Benzyl, -CH₂-OH, H₃CSCH₂CH₂-, HO₂CCH₂-, HO₂CCH₂CH₂-;
- R(51): H, (C₁-C₄)-Alkyl, Phenyl, (CH₂)-Phenyl, wobei der Phenylrest bis zu dreifach substituiert sein kann mit F, Cl, CF₃, Methyl, Methoxy;
sowie deren pharmazeutisch verträgliche Salze. Besonders bevorzugt sind Verbindungen der Formel I 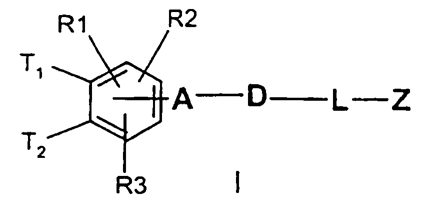 worin ein oder mehrere Rest(e) die folgende Bedeutung hat bzw. haben:
- T1 und T2: unabhängig voneinander gleich 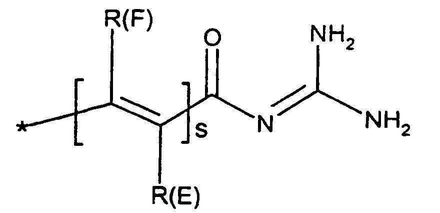 oder Wasserstoff, wobei
T1 und T2 nicht gleichzeitig Wasserstoff sein können,
- L-Z: 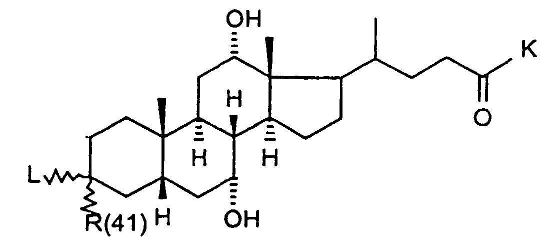
- A: eine Bindung, -CH₂-, CH₂-X-, -X-;
- X: -O-, -CO-, -SO₍₀₋₂₎- ;
- s: Null oder 1;
- D: Phenylen, welches bis zu zweifach substituiert sein kann mit F, Cl, -CF₃, (C₁-C₄)-Alkyl, Hydroxy, Methoxy, -NH₂, NHCH₃, N(CH₃)₂, CH₃SO₂-, H₂NO₂S;
- R(E) F,: (C₁-C₄)-Alkyl, wobei in dem Alkylrest ein, mehrere oder alle Wasserstoffe durch Fluor ersetzt sein können;
- R(F): Wasserstoff;
- R(1), R(2), R(3): unabhängig voneinander Wasserstoff, F, Cl, CN, -SO₂-CH₃, O- (C₀-C₁)-Alkyl-Phenyl, -(C₀-C₁)-Alkyl-Phenyl, wobei der Phenylkern bis zu dreifach substituiert sein kann mit F, Cl, CF₃, Methyl, Methoxy;
- L: (C₁-C₈)-Alkyl , wobei eine odere mehrere (CH₂)-Gruppen ersetzt sein können durch -CH=CH-, -C≡C-, -O-, -NR(47)-, -NR(48)-, -CO-, -SO₂-;
- R(47): Wasserstoff, (C₁-C₄)-Alkyl, R(48)-CO-, Phenyl, (CH₂)-Phenyl;
- R(48): Wasserstoff, (C₁-C₄)-Alkyl, Phenyl, (CH₂)-Phenyl, wobei der Phenylkern bis zu dreifach substituiert sein kann mit F, Cl, CF₃, Methyl, Methoxy;
- R(41): Wasserstoff, -OH;
- K: -OR(51), -NH(R51), -N(R51)₂, -HN-CH₂-CO₂H, -HN-CH₂-CH₂-CO₂H, -HN- CH₂-CH₂-SO₃H, -N(CH₃)CH₂CO₂H, -OKa, wobei Ka ein Kation bedeutet, wie z.B. ein Alkali- oder Erdalkaliion oder ein quartäres Ammoniumion;
- R(51) H,: (C₁-C₄)-Alkyl, Phenyl, (CH₂)-Phenyl, wobei der Phenylrest bis zu dreifach substituiert sein kann mit F, Cl, CF₃, Methyl, Methoxy;
sowie deren pharmazeutisch verträgliche Salze.

Ganz besonders bevorzugt sind Verbindungen der Formel I 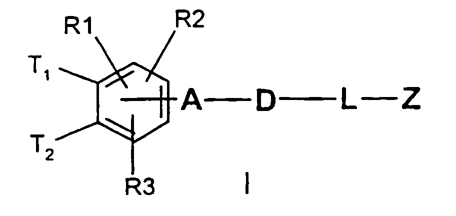 worin ein oder mehrere Rest(e) die folgende Bedeutung hat bzw. haben:
- T1 und T2: unabhängig voneinander 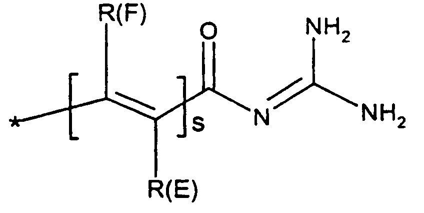 oder Wasserstoff, wobei
T1 und T2 nicht gleichzeitig Wasserstoff sein können,
- L-Z: 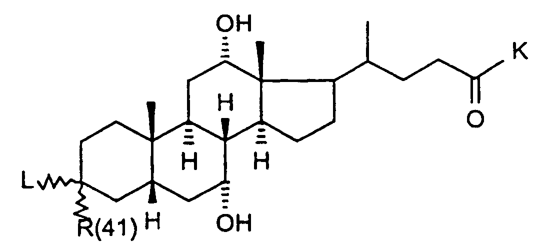
- A: eine Bindung, -O-;
- s: Null oder 1;
- D: Phenylen, welches bis zu zweifach substituiert sein kann mit F, Cl, -CF₃, (C₁-C₄)-Alkyl, Hydroxy, Methoxy, N(CH₃)₂, CH₃SO₂-, H₂NO₂S;
- R(E): (C₁-C₄)-Alkyl;
- R(F): Wasserstoff;
- R(1), R(2), R(3): unabhängig voneinander Wasserstoff F, Cl, CN, -SO₂-CH₃, O- (C₀-C₁)-Alkyl-Phenyl, -(C₀-C₁)-Alkyl-Phenyl, wobei der Phenylkern bis zu dreifach substituiert sein kann mit F, Cl, CF₃, Methyl, Methoxy;
- L: (C₁-C₆)-Alkyl, wobei eine odere mehrere (CH₂)-Gruppen ersetzt sein können durch -CH=CH-, -C≡C-, -O-, -NR(47)-, -CO-, -SO₂-;
- R(47): Wasserstoff, (C₁-C₄)-Alkyl;
- R(41): Wasserstoff, -OH;
- K: -OH, -HN-CH₂-CO₂H, -HN-CH₂-CH₂-CO₂H, -HN-CH₂-CH₂-SO₃H, -N(CH₃)CH₂CO₂H, -OKa, wobei Ka ein Kation bedeutet, wie z.B. ein Alkali- oder Erdalkaliion oder ein quartäres Ammoniumion;
sowie deren pharmazeutisch verträgliche Salze.

Enthält die Verbindung der Formel I ein oder mehrere Asymmetriezentren, so können diese sowohl S als auch R konfiguriert sein. Die Verbindungen können als optische Isomere, als Diastereomere, als Racemate oder als Gemische derselben vorliegen.

Die Doppelbindungsgeometrie der Verbindungen der Formel I kann sowohl E als auch Z sein. Die Verbindungen können als Doppelbindungsisomere im Gemisch vorliegen.

Die bezeichneten Alkylreste können sowohl geradkettig wie verzweigt vorliegen.

Die Erfindung betrifft weiterhin ein Verfahren zur Herstellung der Verbindung I (L-Z = Acetylen-Gallensäurederivat), dadurch gekennzeichnet, daß man eine Verbindung der Formel II, 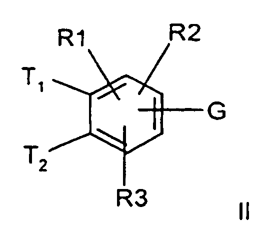 wobei T1, T2, R(1), R(2) und R(3) die oben angegebene Bedeutung haben und G für eine durch L-Z austauschbare Funktionalität steht, in einer dem Fachmann bekannten Art und Weise mit einer Verbindung L-Z der Formel III zur Reaktion bringt. Die Funktionalität G der Verbindung mit der Formel II kann beispielsweise die Bedeutung von Brom oder lod besitzen, da durch Pd(0)-Katalyse in dem Fachmann bekannter Art und Weise die gewünschte C-C-Bindungsknüpfung erzielt 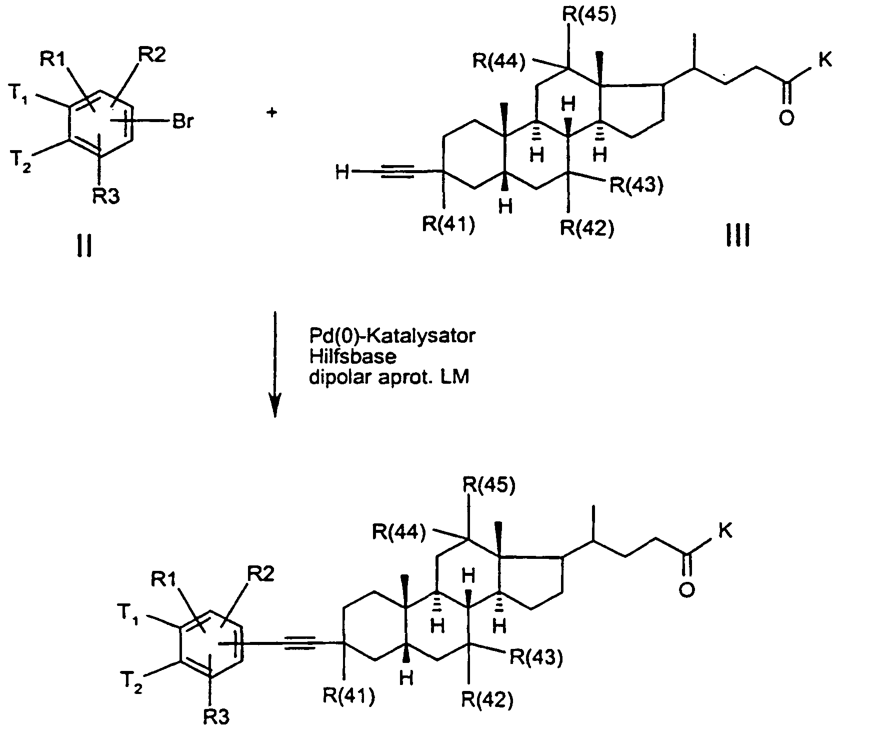 wird.

Die Acetylen-Gallensäurederivate der Formel III werden aus geeigneten Gallensäureketonen IV entsprechend Schema I hergestellt. Diese wiederum können nach literatur-bekannten Verfahren aus Gallensäuren V hergestellt oder käuflich erworben werden. Dazu wird Lithiumacetylid analog zu bekannten Verfahren (US 5,641,767) an Ketogallensäuren des Typs IV addiert. Die so erhaltenen Acetylen-Gallensäurederivate III a werden je nach Art der verwendeten Schutzgruppen in einem Eintopfverfahren, z.B. bei X₃, X₄ = OAcyl, oder in zwei Schritten, z.B. bei X₃, X₄ = THP zu Verbindungen III b entschützt. Die Carbonsäurefunktion von b kann nach unterschiedlichen bekannten Verfahren in geeignete Ester wie z.B. Benzyl-, p-Methoxybenzyl-, Trimethylsilyl oder t-Butylester des Typs III c umgewandelt werden.

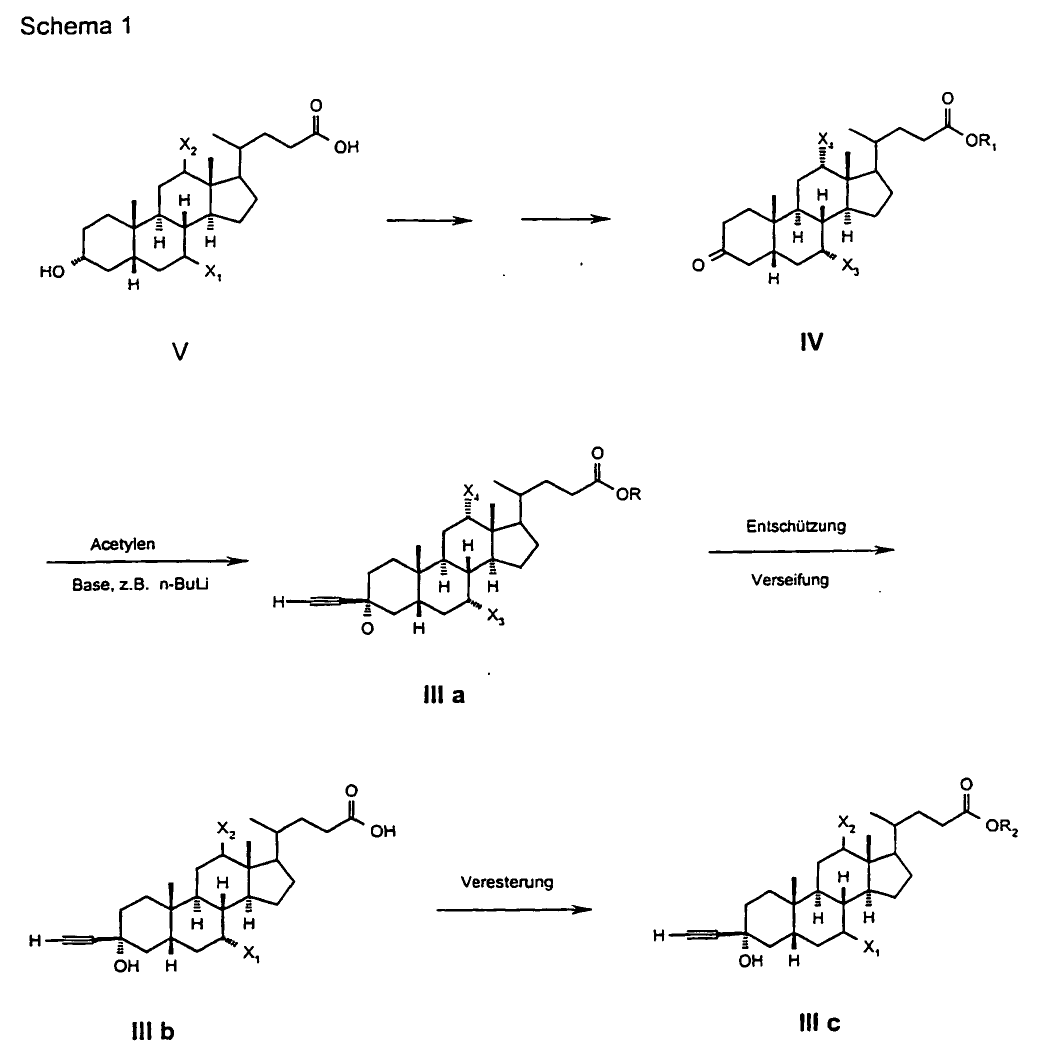

Alkenoylguanidine I sind im allgemeinen schwache Basen und können Säure unter Bildung von Salzen binden. Als Säureadditionssalze kommen Salze aller pharmakologisch verträglichen Säuren in frage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen der Formel I sind substituierte Acylguanidine.

Die erfindungsgemäßen Verbindungen der Formel (I) gelangen auch in das hepatobiliäre System und wirken daher in diesen Geweben. So wird z. B. die Wasserabsorption aus der Gallenblase durch Inhibition des apikalen NHE-Antiports vom Subtyp 3 des Gallenblasenepithels gehemmt, was eine verdünnte Gallenflüssigkeit zur Folge hat.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten.

Im allgemeinen liegt die Tagesdosis im Bereich von 0,1 mg bis 100 mg (typischerweise von 0,1 mg bis 50 mg) pro Tag pro Kilogramm Körpergewicht, z.B. 0,1-10 mg/kg/Tag. Tabletten oder Kapseln können beispielsweise von 0,01 bis 100 mg, typischerweise von 0,02 bis 50 mg enthalten. Im Falle pharmazeutisch verträglicher Salze beziehen sich die vorgenannten Gewichtsangaben auf das Gewicht des vom Salz abgeleiteten Aminopropanol-lons. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

### Experimenteller Teil

### Liste der Abkürzungen:

- CH₂Cl₂: Dichlormethan
- DCC: Dicyclohexyl-carbodiimid
- DCI: Desorption-Chemicallonisation
- DIP: Di-*i*-propylether
- DME: Dimethoxyethan
- DMF: N,N-Dimethylformamid
- EE: Ethylacetat (EtOAc)
- El: electron impact
- eq.: Äquivalent
- ES: Elektronenspray
- FAB: Fast Atom Bombardment
- HEP: n-Heptan
- HOBT: Benzotriazol-1-ol
- KOtBu: Kalium-2-methyl-propan-2-olat
- MeOH: Methanol
- mp: Schmelzpunkt
- MTB: *t*-Butylmethylether
- RT: Raumtemperatur
- THF: Tetrahydrofuran
- TOTU: O[(Cyano(ethoxycarbonyl)methylen)amino]-1,1,3,3-tetramethyluroniumtetrafluorborat

### Beispiel 1 4-{3-[2-(2-{4-[2,6-Difluor-4-(3-guanidino-2-methyl-3-oxo-propenyl)-phenoxy]-phenyl}-acetylamino)-ethoxy]-7,12-dihydroxy-10,13-dimethyl-

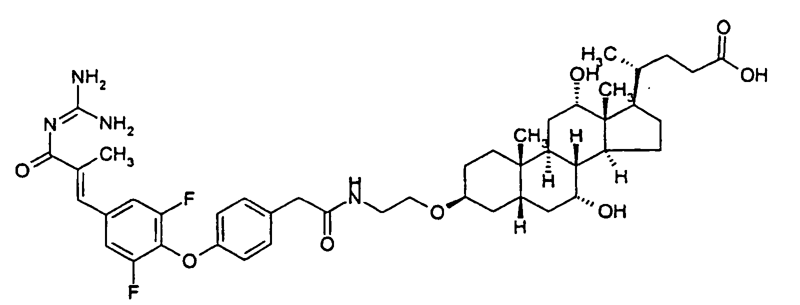

hexadecahydro-cyclopenta[a]phenanthren-17-yl}-pentansäure
a) 4-(7,12-Dihydroxy-3-methanesulfonyloxy-10,13-dimethyl-hexadecahydrocyclopenta[a]phenanthren-17-yl)-pentansäure
   100 g Cholsäure werden in 500 ml Pyridin gelöst und bei 0°C 23,1 ml Mesylchlorid über einen Zeitraum von 30 Minuten zugetropft. 3 Stunden lang wird bei RT gerührt, anschließend bei 0°C auf eine Lösung von 400 ml H₂SO₄ in 3 l Wasser gegossen und 4 mal mit je 750 ml EE extrahiert. Über Na₂SO₄ wird getrocknet und das Solvens im Vakuum entfernt. Der Rückstand wird mit Diisopropylether zur Kristallisation gebracht und man erhält 117,1 g; mp 121°C (unter Zersetzung).
   R_{f}(EE/HEP/Essigsäure 5:5:1) = 0.31 MS (FAB) : 487 (M+H)⁺
b) 4-[7,12-Dihydroxy-3-(2-hydroxy-ethoxy)-10,13-dimethyl-hexadecahydrocyclopenta[a]phenanthren-17-yl]-pentansäure-methylester
   116 g 4-(7,12-Dihydroxy-3-methanesulfonyloxy-10,13-dimethyl-hexadecahydrocyclopenta[a]phenanthren-17-yl)-pentansäure sowie 130 ml Triethylamin werden in 650 ml Glycol gelöst und 3 Stunden bei 100°C sowie 7,5 Stunden bei 115°C gerührt. Das Reaktionsgemisch wird bei 0°C auf eine Lösung von 400 ml H₂SO₄ in 3 l Wasser gegossen und 7 mal mit je 750 ml EE extrahiert. Über Na₂SO₄ wird getrocknet und das Solvens im Vakuum entfernt. Man erhält das Zwischenprodukt ZWP.
   Bei 0°C werden 130 ml Acetylchlorid zu 900 ml Methanol getropft. Dann wird eine Lösung von ZWP in 400 ml zugegeben und 6 Stunden bei RT gerührt. 60 Stunden wird bei RT stehen gelassen, dann auf 2.6 l Wasser gegossen und 8 mal mit je 500 ml Diisopropylether (DIP) extrahiert. Die organische Phase wird anschließend noch 6 mal mit je 600 ml einer halbgesättigten wäßrigen NaHCO₃-Lösung gewaschen. Über Na₂SO₄ wird getrocknet und das Solvens im Vakuum entfernt. Chromatographie an Kieselgel mit EE liefert 32 g eines harzähnlichen Feststoffs.
   R_{f}(EE) = 0.19 MS (FAB): 467 (M+H)⁺
c) 4-{3-[2-(1,3-Dioxo-1,3-dihydro-isoindol-2-yl)-ethoxy]-7,12-dihydroxy-10,13-dimethyl-hexadecahydro-cyclopenta[a]phenanthren-17-yl}-pentansäuremethylester
   1.5 g 4-[7,12-Dihydroxy-3-(2-hydroxy-ethoxy)-10,13-dimethyl-hexadecahydrocyclopenta[a]phenanthren-17-yl]-pentansäure-methylester, 950 mg Triphenylphosphin und 550 mg Phthalimid werden in 26 ml THF auf 45°C erwärmt und bei dieser Temperatur 1,14 ml Azodicarbonsäurediethylester zugetropft. 2 Stunden wird bei 45°C gerührt, anschließend das Reaktionsgemisch in 200 ml einer halbkonzentrierten wäßrigen NaHCO₃-Lösung gegossen und 3 mal mit je 200 ml EE extrahiert. Über Na₂SO₄ wird getrocknet und das Solvens im Vakuum entfernt. Chromatographie an Kieselgel mit *t*-Butylmethylether (MTB) liefert 1.76 g eines zähen Öls.
   R_{f}(EE) = 0.60 MS (FAB) : 602 (M+Li)⁺
d) 4-[3-(2-Amino-ethoxy)-7,12-dihydroxy-10,13-dimethyl-hexadecahydrocyclopenta[a]phenanthren-17-yl]-pentansäure-methylester
   1.7 g 4-{3-[2-(1,3-Dioxo-1,3-dihydro-isoindol-2-yl)-ethoxy]-7,12-dihydroxy-10,13-dimethyl-hexadecahydro-cyclopenta[a]phenanthren-17-yl}-pentansäure-methylester sowie 0.52 ml Hydrazinhydrat (80%) werden in 14 ml Methanol gelöst und 3 Stunden unter Rückfluß gekocht. Anschließend wird auf 40°C abgekühlt und das Reaktionsgemisch mit 8.7 ml einer 2N wäßrigen HCl-Lösung versetzt. 30 Minuten wird bei 40°C nachgerührt, anschließend die flüchtigen Bestandteile im Vakuum entfernt. Chromatographie an Kieselgel mit Aceton/Wasser 10:1 liefert 540 mg harzähnlichen Feststoffs.
   R_{f}(Aceton/Wasser 10:1) = 0.06 MS (FAB) : 466 (M+H)⁺
e) 4-(7,12-Dihydroxy-3-{2-[2-(4-hydroxy-phenyl)-acetylamino]-ethoxy}-10,13-dimethyl-hexadecahydro-cyclopenta[a]phenanthren-17-yl)-pentansäuremethylester
   700 mg 4-[3-(2-Amino-ethoxy)-7,12-dihydroxy-10,13-dimethyl-hexadecahydrocyclopenta[a]phenanthren-17-yl]-pentansäure-methylester, 230 mg 4-Hydroxyphenylessigsäure und 305 mg HOBT werden in 10 ml THF gelöst und bei 0°C eine Lösung von 342 mg DCC in 10 ml THF zugespritzt. Eine Stunde wird bei 0°C und anschließend 25 Stunden bei RT gerührt. Der Niederschlag wird abfiltriert, das Filtrat mit 150 ml einer 10% wäßrigen NaHCO₃-Lösung verdünnt und drei mal mit je 100 ml EE extrahiert. Über Na₂SO₄ wird getrocknet und das Solvens im Vakuum entfernt. Chromatographie an Kieselgel mit EE/MeOH 10:1 liefert 660 mg eines farblosen Öls.
   R_{f}(EE/MeOH 10:1) = 0.38 MS (FAB): 600 (M+H)⁺
f) 4-(7,12-Dihydroxy-3-{2-[2-(4-hydroxy-phenyl)-acetylamino]-ethoxy}-10,13-dimethyl-hexadecahydro-cyclopenta[a]phenanthren-17-yl)-pentansäure
   620 mg 4-(7,12-Dihydroxy-3-{2-[2-(4-hydroxy-phenyl)-acetylamino]-ethoxy}-10,13-dimethyl-hexadecahydro-cyclopenta[a]phenanthren-17-yl)-pentansäure werden in 10 ml MeOH gelöst und bei RT 3,1 ml einer 1 N wäßrigen NaOH-Lösung zugegeben. 25 Stunden wird bei RT gerührt, mit 150 ml einer 2N wäßrigen HCl-Lösung versetzt und 3 mal mit je 100 ml EE extrahiert. Über Na₂SO₄ wird getrocknet und das Solvens im Vakuum entfernt. Man erhält 630 mg eines mit geringen Mengen Essigsäure verunreinigten Produktes, das ohne weitere Reinigung umgesetzt wird.
   R_{f}(EE/MeOH 5:1) = 0.30 MS (ES) : 586 (M+H)⁺
g) 2-Methyl-3-(3,4,5-trifluoro-phenyl)-acrylsäure-ethylester
   4.3 ml 2-Phosphonopropionsäure-triethylester werden in 30 ml wasserfreiem THF gelöst und bei 0°C 12.5 ml einer 1.6 N Lösung von n-Butyllithium in Hexan zugetropft. 15 Minuten wird bei RT gerührt und anschließend eine Lösung von 3.2 g 3,4,5-Trifluorbenzaldehyd in 8 ml wasserfreiem THF zugetropft. Eine Stunde wird bei RT gerührt und 16 Stunden bei RT stehen gelassen. Das Reaktionsgemisch wird mit 300 ml Wasser verdünnt, 30 ml einer gesättigten wäßrigen Na₂CO₃-Lösung zugegeben und 3 mal mit je 100 ml EE extrahiert. Über Na₂SO₄ wird getrocknet und das Solvens im Vakuum entfernt. Chromatographie an Kieselgel mit EE/HEP 1:8 liefert 3.8 g farbloser Kristalle; mp 54°C.
   R_{f}(EE/HEP 1:8) = 0.35 MS (DCI): 245 (M+H)⁺
h) 3-{4-[4-({2-[17-(3-Carboxy-1-methyl-propyl)-7,12-dihydroxy-10,13-dimethylhexadecahydro-cyclopenta[a]phenanthren-3-yloxy]-ethylcarbamoyl}-methyl)-phenoxy]-3,5-difluoro-phenyl}-2-methyl-acrylsäure-ethylester
   610 mg 4-(7,12-Dihydroxy-3-{2-[2-(4-hydroxy-phenyl)-acetylamino]-ethoxy}-10,13-dimethyl-hexadecahydro-cyclopenta[a]phenanthren-17-yl)-pentansäure, 232 mg 2-Methyl-3-(3,4,5-trifluoro-phenyl)-acrylsäure-ethylester sowie 393 mg K₂CO₃ werden in 5 ml DMF (wasserfrei) suspendiert und 160 Minuten bei 150°C gerührt. Nach dem Abkühlen wird das Reaktionsgemisch mit 150 ml einer 2N wäßrigen HCl-Lösung aufgenommen und drei mal mit je 100 ml EE extrahiert. Über Na₂SO₄ wird getrocknet und das Solvens im Vakuum entfernt. Chromatographie an Kieselgel mit EE/MeOH 10:1 liefert 480 mg harzähnlichen Produkts.
   R_{f}(EE/MeOH 10:1) = 0.27 MS (ES): 810 (M+H)⁺
i) 4-{3-[2-(2-{4-[2,6-Difluor-4-(3-guanidino-2-methyl-3-oxo-propenyl)-phenoxy]-phenyl}-acetylamino)-ethoxy]-7,12-dihydroxy-10,13-dimethyl-hexadecahydrocyclopenta[a]phenanthren-17-yl}-pentansäure
   311 mg Guanidin-Hydrochlorid werden in 3 ml DMF (wasserfrei) gelöst und bei RT eine Lösung von 332 mg KOtBu in 3 ml DMF (wasserfrei) zugegeben. Eine Stunde wird bei RT gerührt, anschließend eine Lösung von 480 mg 3-{4-[4-({2-[17-(3-Carboxy-1-methyl-propyl)-7,12-dihydroxy-10,13-dimethyl-hexadecahydrocyclopenta[a]phenanthren-3-yloxy]-ethylcarbamoyl}-methyl)-phenoxy]-3,5-difluorophenyl)-2-methyl-acrylsäure-ethylester in 3 ml DMF (wasserfrei) addiert. 65 Stunden wird bei RT gerührt und anschließend das Reaktionsgemisch mit 200 ml Wasser verdünnt. Mit 4 N wäßriger HCl-Lösung wird auf pH = 7 gestellt und drei mal mit je 150 ml EE extrahiert. Über Na₂SO₄ wird getrocknet und das Solvens im Vakuum entfernt.

Chromatographie an Kieselgel mit CH₂Cl₂/MeOH/Aceton/Wasser/Essigsäure 64:8:8:1:1 liefert 230 mg eines amorphen Feststoffs.
R_{f}(CH₂Cl₂/MeOH/Aceton/Wasser/Essigsäure 64:8:8:1:1) = 0.27
MS (ES) : 823 (M+H)⁺

Die Verbindung des Beispiels 2 wurde analog Beispiel 1 aus 4-(3-Amino-7,12-dihydroxy-10,13-dimethyl-hexadecahydro-cyctopenta[a]phenanthren-17-yl)-pentansäure hergestellt:

### Beispiel 2 4-[3-(2-{4-[2,6-Difluoro-4-(3-guanidino-1-methyl-3-oxo-propenyl)-phenoxy]-phenyl}- acetylamino)-7,12-dihydroxy-10,13-dimethyl-hexadecahydrocyclopenta[a]phenanthren-17- yl]-pentansäure

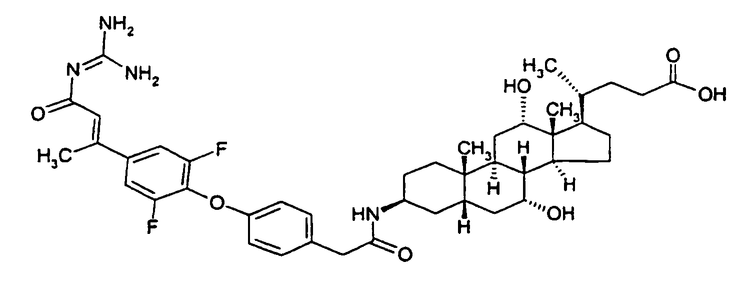

mp 190°C (unter Zersetzung)
R_{f}(CH₂Cl₂/MeOH/Wasser/Essigsäure 32:8:1:1) = 0.36 MS (ES) : 779 (M+H)⁺

### Beispiel 3 4-(3-{2-[(4'-Guanidinocarbonyl-biphenyl-3-carbonyl)-amino]-ethoxy}-7,12-dihydroxy-10,13- dimethyl-hexadecahydro-cyclopenta[a]phenanthren-17-yl)-pentansäure

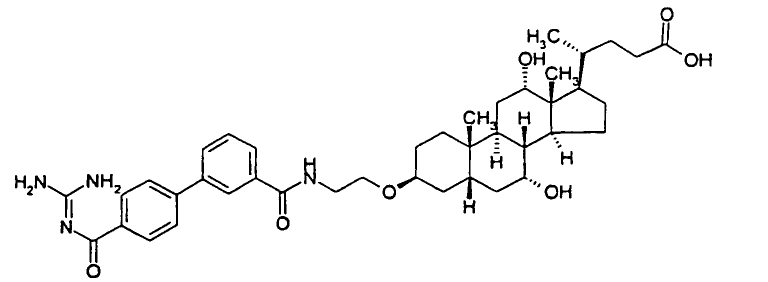

a) 4-{3-[2-(3-Brom-benzoylamino)-ethoxy]-7,12-dihydroxy-10,13-dimethylhexadecahydro-cyclopenta[a]phenanthren-17-yl}-pentansäure-methylester
   932 mg 4-[3-(2-Amino-ethoxy)-7,12-dihydroxy-10,13-dimethyl-hexadecahydrocyclopenta[a]phenanthren-17-yl]-pentansäure-methylester (Beispiel 1 d) und 402 mg 3-Brombenzoesäure werden in 20 ml wasserfreiem DMF gelöst und bei 0°C zunächst 656 mg O-[(Cyano(ethoxycarbonyl)methylen)amino]-1,1,3,3-tetramethyluroniumtetrafluorborat (TOTU) (Proceedings of the 21^{st} European Peptide Symposium, Peptides 1990, Editors E. Giralt and D. Andreu, Escom, Leiden, 1991) und dann 760µL N-Ethylmorpholin zugegeben. 20.5 Stunden wird bei RT gerührt und das Reaktionsgemisch anschließend auf 100 ml einer 10% wäßrigen NaHCO₃-Lösung gegossen. 3 mal wird mit je 150 ml EE extrahiert. Über Na₂SO₄ wird getrocknet und das Solvens im Vakuum entfernt. Chromatographie an Kieselgel mit EE liefert 610 mg eines zähen Öls.
   R_{f}(EE) = 0.33 MS (ES) : 649 (M+H)⁺
b) 4-{3-[2-(3-Brom-benzoylamino)-ethoxy]-7,12-dihydroxy-10,13-dimethylhexadecahydro-cyclopenta[a]phenanthren-17-yl}-pentansäure
   600 mg 4-{3-[2-(3-Brom-benzoylamino)-ethoxy]-7,12-dihydroxy-10,13-dimethylhexadecahydro-cyclopenta[a]phenanthren-17-yl}-pentansäure-methylester werden in 19 ml Methanol gelöst und 4.6 ml einer 1 N wäßrigen NaOH-Lösung zugegeben. 6 Stunden wird bei RT gerührt, anschließend in 150 ml einer 2N wäßrigen HCl-Lösung gegossen und 4 mal mit je 150 ml MTB extrahiert. Über Na₂SO₄ wird getrocknet und das Solvens im Vakuum entfernt. Man erhält 550 mg eines zähen Öls, das so weiter eingesetzt wird.
   R_{f}(EE/MeOH 5:1) = 0.46 MS (ES): 635 (M+H)⁺
c) 3'-{2-[17-(3-Carboxy-1-methyl-propyl)-7,12-dihydroxy-10,13-dimethylhexadecahydro- cyclopenta[a]phenanthren-3-yloxy]-ethylcarbamoyl}-biphenyl-4-carbonsäure-ethylester
   540 mg 4-{3-[2-(3-Brom-benzoylamino)-ethoxy]-7,12-dihydroxy-10,13-dimethylhexadecahydro-cyclopenta[a]phenanthren-17-yl}-pentansäure, 4-Dihydroxyboryl-benzoesäure-ethylester, 9.6 mg Pd(II)-acetat und 22.3 mg Triphenylphosphin werden in 5.1 ml Toluol sowie 1.4 ml Ethanol gelöst und 425 µL einer 2N wäßrigen Na₂CO₃-Lösung zugegeben. 3 Stunden wird unter Rückfluß gekocht, anschließend weitere 33.8 mg 4-Dihydroxyboryl-benzoesäure-ethylester und 425 µL einer 2N wäßrigen Na₂CO₃-Lösung zugegeben und nochmals 1 Stunde 35 Minuten unter Rückfluß gekocht. Dann werden erneut 33.8 mg 4-Dihydroxyboryl-benzoesäure-ethylester und 425 µL einer 2N wäßrigen Na₂CO₃-Lösung zugegeben und nochmals 4 Stunden 40 Minuten unter Rückfluß gekocht. Nach dem Abkühlen wird das Reaktionsgemisch mit 150 ml einer 2N wäßrigen HCl-Lösung aufgenommen und 4 mal mit je 100 ml EE extrahiert. Über Na₂SO₄ wird getrocknet und das Solvens im Vakuum entfernt. Chromatographie an Kieselgel mit MTB/2% Essigsäure liefert 510 mg eines farblosen Schaums.
   R_{f}(MTB/2% Essigsäure) = 0.21 MS (ES) : 635 (M+H)⁺
d) 4-(3-{2-[(4'-Guanidinocarbonyl-biphenyl-3-carbonyl)-amino]-ethoxy}-7,12-dihydroxy-10,13- dirnethyl-hexadecahydro-cyclopenta[a]phenanthren-17-yl)-pentansäure
   115 mg Guanidin-Hydrochlorid werden in 2 ml wasserfreiem DMF gelöst und bei RT eine Lösung von 122 mg Kalium-*t*-butylat in 2 ml wasserfreiem DMF addiert. Eine Stunde wird bei RT nachgerührt und anschließend eine Lösung von 150 mg 3'-{2-[17-(3-Carboxy-1-methyl-propyl)-7,12-dihydroxy-10,13-dimethyl-hexadecahydrocyclopenta[a]phenanthren-3-yloxy]-ethylcarbamoyl}-biphenyl-4-carbonsäure-ethylester in 3 ml wasserfreiem DMF zugegeben. 4 Tage und 2 Stunden wird bei RT gerührt, anschließend das Reaktionsgemisch mit 35 ml Wasser versetzt und mit wäßriger HCl-Lösung auf pH=6.5 gestellt. 1 Stunde wird nachgerührt und der Hauptteil des Produktes ausgefällt. Das Filtrat wird 3 mal mit je 100 ml EE extrahiert. Über Na₂SO₄ wird getrocknet und das Solvens im Vakuum entfernt. Chromatographie an Kieselgel mit CH₂Cl₂/MeOH/Aceton/Wasser/Essigsäure 64:8:8:1:1 liefert 60 mg eines amorphen Feststoffs.
   R_{f}(CH₂Cl₂/MeOH/Aceton/Wasser/Essigsäure 64:8:8:1:1) = 0.13 MS (ES) : 635 (M+H)⁺

Die Wasserabsorption aus der Gallenblase wird durch Inhibition des apikalen NHE-Antiports vom Subtyp 3 des Gallenblasenepithels gehemmt, was eine verdünnte Gallenflüssigkeit zur Folge hat. Diese verdünnte Gallenflüssigkeit verhindert eine Gallensteinbildung und bewirkt bei bereits vorhandenen Gallensteinen deren Auflösung.

Die Inhibition des NHE-Antiports vom Subtyp 3 durch die erfindungsgemäßen Verbindungen der Formel I wurde mittels folgendem Test bestimmt.

Die meisten der molekularbiologischen Techniken folgen Protokollen aus den Werken "Current Protocols in Molecular Biology (eds. Ausubel, F.M., Brent, R., Kingston, R.E., Moore, D.D., Seidman, J.G., Smith, J.A. und Struhl, K.; John Wiley & Sons)" bzw: "Molecular Cloning: A Laboratory Manual (Sambrock, J., Fritsch, E.F. und Maniatis, T.; Cold Spring Harbor Laboratory Press (1989))".

Im Rahmen unserer Arbeiten wurden stabil transfizierte Zellinien erzeugt, die jeweils einen der folgenden NHE-Subtypen exprimieren: NHE1 des Menschen (Sardet et al.; Cell 56, 271-280 (1989), NHE2 des Kaninchens (Tse et al.; J. Biol. Chem. 268, 11917 - 11924 (1993)) bzw. NHE3 der Ratte (Orlowski et al.; J. Biol. Chem. 267, 9331 - 9339 (1992)), NHE3 des Menschen (Braut et al.; Am. J. Physiol. 269, 198-206 (1995)).

Die von Prof. Pouysségur erhaltenen cDNA-Klone der jeweiligen NHE-Subtypen wurden nach Anfügen geeigneter Linkersequenzen so in das Expressionsplasmid pMAMneo (erhältlich z. B. über CLONTECH, Heidelberg) einkloniert, daß die NHE1-Erkennungssequenz des Plasmids etwa 20 - 100 Basenpaare vor dem Startcodon des jeweiligen NHE-Subtyps liegt und die gesamte codierende Sequenz in dem Konstrukt vorhanden ist.

Mit der sog. "Calciumphosphat-Methode" (beschrieben in Kapitel 9.1 von "Current Protocols in Molecular Biology") wurde die NHE-defiziente Zellinie LAP1 (Franchi et al.; Proc. Natl. Acad. Sci. USA 83, 9388 - 9392 (1986)) mit den Plasmiden, die die jeweiligen codierenden Sequenzen der NHE-Subtypen enthalten, transfiziert. Nach Selektion auf transfizierte Zellen über Wachstum in G418-haltigem Medium (nur

Zellen, die durch Transfektion ein neo-Gen erhalten haben, können unter diesen Bedingungen überleben) wurde auf eine funktionelle NHE-Expression selektioniert. Dazu wurde die von Sardet beschriebene "Acid Load"-Technik verwendet (Sardet et al.; Cell 56, 271 - 280 (1989)). Zellen, die einen funktionsfähigen NHE-Subtypen exprimieren, können auch bei Abwesenheit von CO₂ und HCO₃- die bei diesem Test vorgenommene Ansäuerung kompensieren, untransfizierte LAP1-Zellen dagegen nicht. Nach mehrmaliger Wiederholung der "Acid Load" Selektion wurden die überlebenden Zellen in Mikrotiterplatten so ausgesät, daß statistisch eine Zelle pro Well vorkommen sollte. Unter dem Mikroskop wurde nach etwa 10 Tagen überprüft, wie viele Kolonien pro Well wuchsen. Zellpopulationen aus Einzelkolonien wurden dann mit dem XTT-Proliferation Kit (Boehringer Mannheim) hinsichtlich ihrer Überlebensfähigkeit nach "Acid Load" untersucht. Die besten Zellinien wurden für die weiteren Tests verwendet und zur Vermeidung eines Verlustes der transfizierten Sequenz unter ständigem Selektionsdruck in G418-haltigem Medium kultiviert. Zur Bestimmung von IC₅₀-Werten für die Hemmung der einzelnen NHE-Subtypen durch spezifische Substanzen wurde ein von S. Faber entwickelter Test (Faber et al.; Cell. Physiol. Biochem. 6, 39 - 49 (1996)), der auf der "Acid Load" Technik beruht, leicht abgewandelt.

In diesem Test wurde die Erholung des intrazellulären pHs (pHi) nach einer Ansäuerung ermittelt, die bei funktionsfähigem NHE auch unter bicarbonatfreien Bedingungen einsetzt. Dazu wurde der pHi mit dem pH-sensitiven Fluoreszenzfarbstoff BCECF (Calbiochem, eingesetzt wird die Vorstufe BCECF-AM) bestimmt. Die Zellen wurden zunächst mit BCECF beladen. Die BCECF-Fluoreszenz wurde in einem "Ratio Fluorescence Spectrometer" (Photon Technology International, South Brunswick, N.J., USA) bei Anregungswellenlängen von 505 und 440 nm und einer Emissionswellenlänge von 535 nm bestimmt und mittels Kalibrierungskurven in den pHi umgerechnet. Abweichend von dem beschriebenen Protokoll wurden die Zellen bereits bei der BCECF-Beladung in NH₄Cl-Puffer (pH 7,4) inkubiert (NH₄Cl-Puffer: 115 mM NaCl, 20 mM NH₄Cl, 5 mM KCI, 1 mM CaCl₂, 1 mM MgSO₄, 20 mM Hepes, 5 mM Glucose, 1 mg/ml BSA; mit 1 M NaOH wird ein pH von 7,4 eingestellt). Die intrazelluläre Ansäuerung wurde durch Zugabe von 975 µl eines NH₄Cl-freien Puffers zu 25 µl Aliquots der in NH₄Cl-Puffer inkubierten Zellen induziert. Die nachfolgende Geschwindigkeit der pH-Erholung wurde bei NHE1 2 Minuten, bei NHE2 5 Minuten und bei NHE3 3 Minuten registriert. Für die Berechnung der inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Na⁺-haltigem Puffer inkubiert (133,8 mM NaCl, 4,7 mM KCI, 1,25 mM CaCl₂, 1,25 mM MgCl₂, 0,97 mM Na₂HPO₄, 0,23 mM NaH₂PO₄, 5 mM Hepes, 5 mM Glucose, mit 1 M NaOH wird ein pH von 7,0 eingestellt). Für die Bestimmung des 0%-Wertes wurden die Zellen in einem Na⁺-freien Puffer inkubiert (133,8 mM Cholinchlorid, 4,7 mM KCI, 1,25 mM CaCl₂, 1,25 mM MgCl₂, 0,97 mM K₂HPO₂, 0,23 mM KH₂PO₄, 5 mM Hepes, 5 mM Glucose, mit 1 M NaOH wird ein pH von 7,0 eingestellt). Die zu testenden Substanzen wurden in dem Na⁺-haltigem Puffer angesetzt. Die Erholung des intrazellulären pHs bei jeder getesteten Konzentration einer Substanz wurde in Prozent der maximalen Erholung ausgedrückt. Aus den Prozentwerten der pH-Erholung wurde mittels des Programms SigmaPlot der IC₅₀-Wert der jeweiligen Substanz für die einzelnen NHE-Subtypen berechnet.

Ergebnisse zur Inhibition des humanen Na⁺/H⁺-Exchangers (Subtyp 3; NHE-3):

| Beispiel ( siehe exp. Teil ) | Restaktivität bei 30 µM |
|---|---|
| 1 | 17% |
| 2 | 10% |
| 3 | 65% |

Aus den gemessenen Daten ist ersichtlich, daß die erfindungsgemäßen Verbindungen der Formel I den NHE-Antiport bis zu 65% hemmen.

## Patentansprüche

1. Verbindungen der Formel I 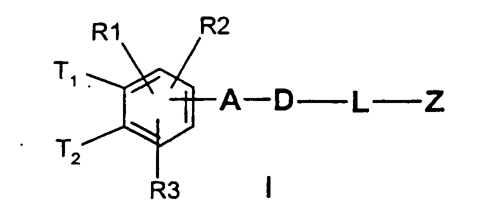 worin bedeuten:
T1 und T2 unabhängig voneinander 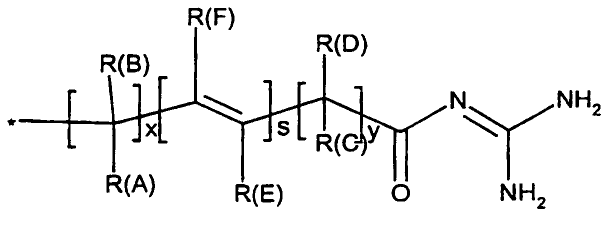 oder Wasserstoff, wobei
T1 und T2 nicht gleichzeitig Wasserstoff sein können;
Z 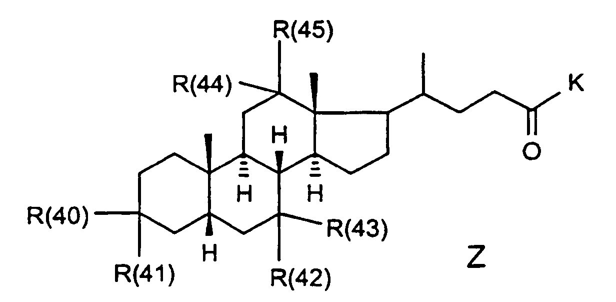
A eine Bindung, (C₁-C₄)-alkyl, (C₀-C₄)-alkyl-X;
X -O-, -CO-, -CH[OH]-, -CH[OCH₃]-, -SO₍₀₋₂₎- oder -NH-, -N(CH₃)-;
D Phenylen, welches nicht substituiert oder substituiert ist mit 1 - 3 Substituenten ausgewählt aus der Gruppe bestehend aus F, Cl, -CF₃, (C₁-C₄)-Alkyl, Hydroxy, Methoxy, -NH₂, NHCH₃, N(CH₃)₂, CH₃SO₂- und H₂NO₂S-;
R(A), R(B), R(C), R(D), R(E), R(F) unabhängig voneinander Wasserstoff, F, Cl, Br, I, CN, OH, OR(6), NR(7)R(8), (C₁-C₈)-Alkyl, O-(C₁-C₁₂)-Alkyl, (C₃-C₈)-Cycloalkyl, wobei in den Alkylresten ein, mehrere oder alle Wasserstoffe durch Fluor ersetzt sein können;
R(6) (C₃-C₆)-Alkenyl, (C₃-C₈)-Cycloalkyl, Phenyl oder Benzyl, wobei der Phenylkern bis zu dreifach substituiert sein kann mit F, Cl, CF₃, Methyl, Methoxy, NR(9)R(10);
R(9), R(10) unabhängig voneinander H, (C₁-C₄)-Alkyl oder (C₁-C₄)-Perfluoralkyl;
R(7) und R(8) unabhängig voneinander (C₁-C₄)-Alkyl, (C₃-C₆)-Alkenyl, (C₃-C₈)-Cycloalkyl, Phenyl oder Benzyl, wobei der Phenylkern bis zu dreifach substituiert sein kann mit F, Cl, CF₃, Methyl, Methoxy, NR(9)R(10); oder
R(7) und R(8) bilden gemeinsam eine Kette von 4 oder 5 Methylengruppen, von denen eine CH₂-Gruppe durch Sauerstoff, Schwefel, NH, N-CH₃ oder N-Benzyl ersetzt sein kann;
s Null oder 1,
x Null, 1 oder 2;
y Null, 1 oder 2;
R(1), R(2), R(3) unabhängig voneinander Wasserstoff, F, Cl, Br, I, CN, -(C=O)- N=C(NH₂)₂, -SO₍₀₋₁₎-(C₁-C₈)-Alkyl , O-(C₀-C₄)-Alkyl-Phenyl, -(C₀-C₄)-Alkyl-Phenyl, wobei der Phenylkern bis zu dreifach substituiert sein kann mit F, Cl, CF₃, Methyl, Methoxy, -(C₀-C₄)-Alkyl-NR(21)R(22); (C₁-C₈)-Alkyl, O-(C₁-C₁₂)-Alkyl, (C₃-C₈)-Cycloalkyl, wobei in den Alkylresten ein, mehrere oder alle Wasserstoffe durch Fluor ersetzt sein können;
R(21), R(22) unabhängig voneinander H, (C₁-C₄)-Alkyl;
L (C₁-C₁₅)-Alkyl , wobei eine oder mehrere (CH₂)-Gruppen ersetzt sein können durch -CH=CH-, -C≡C-, -O-, -NR(47)-, -NR(48)-, -CO-, -SO₂-;
R(47) Wasserstoff, (C₁-C₈)-Alkyl, R(48)-CO-, Phenyl, Benzyl;
R(48) Wasserstoff, (C₁-C₈)-Alkyl, Phenyl, (CH₂)-Phenyl, wobei der Phenylkern bis zu dreifach substituiert sein kann mit F, Cl, CF₃, Methyl, Methoxy;
R(40) bis R(45) unabhängig voneinander H, -O R(50), -S R(50), NH R(50), -N R(50)₂, -O-(CO)- R(50), -S-(CO)- R(50), -NH-(CO)- R(50), -O-PO-(O R(50))-O R(50), -O-(SO₂)-O R(50), -R(50), eine Bindung zu L; oder
R(40) und R(41), R(42) und R(43), R(44) und R(45) jeweils gemeinsam der Sauerstoff einer Carbonylgruppe;
wobei immer genau einer der Reste die Bedeutung einer Bindung zu L hat;
R(50) Wasserstoff, (C₁-C₄)-Alkyl, Phenyl, (CH₂)-Phenyl, wobei der Phenylkern bis zu dreifach substituiert sein kann mit F, Cl, CF₃, Methyl, Methoxy;
K -OR(51), -NH(R51), -N(R51)₂, -HN-CH₂-CH₂-CO₂H, -HN-CH₂-CH₂-SO₃H, -N(CH₃)CH₂CO₂H, -HN-CH(R46)CO₂H, -OKa, wobei Ka ein Kation bedeutet, wie z.B. ein Alkali- oder Erdalkaliion oder ein quartäres Ammoniumion;
R(46) Wasserstoff, C₁-C₄-Alkyl, Benzyl, -CH₂-OH, H₃CSCH₂CH₂-, HO₂CCH₂-, HO₂CCH₂CH₂-;
R(51) H, (C₁-C₄)-Alkyl, Phenyl, (CH₂)-Phenyl, wobei der Phenylrest bis zu dreifach substituiert sein kann mit F, Cl, CF₃, Methyl, Methoxy;
sowie deren pharmazeutisch verträgliche Salze und physiologisch funktionelle Derivate.

2. Verbindungen der Formel I, gemäß Anspruch 1, **dadurch gekennzeichnet, daß** darin bedeuten
T1 und T2 unabhängig voneinander 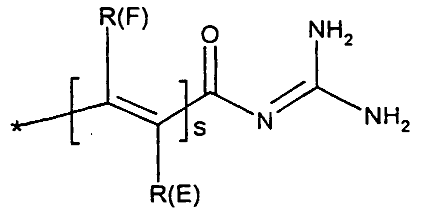 oder Wasserstoff, wobei
T1 und T2 nicht gleichzeitig Wasserstoff sein können;
L-Z 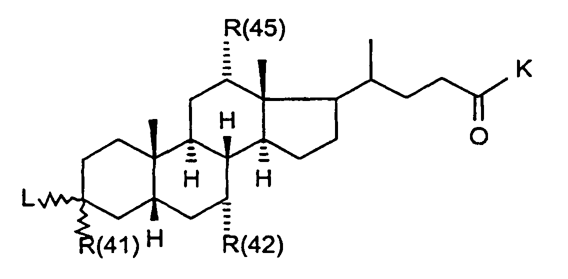
A eine Bindung, -CH₂- , CH₂-X-;
X -O-, -CO-, -CH[OH]-, -CH[OCH₃]-, -SO₍₀₋₂₎- oder -NH-, N(CH₃)-;
s Null oder 1;
D Phenylen, welches bis zu zweifach substituiert sein kann mit F, Cl, -CF₃, (C₁-C₄)-Alkyl, Hydroxy, Methoxy, -NH₂, NHCH₃, N(CH₃)₂ , CH₃SO₂-, H₂NO₂S-;
R(E) F, Cl, CN, OR(12), (C₁-C₄)-Alkyl, O-(C₁-C₄)-Alkyl, (C₃-C₆)-Cycloalkyl, wobei in den Alkylresten ein, mehrere oder alle Wasserstoffe durch Fluor ersetzt sein können;
R(6) (C₃-C₆)-Alkenyl, (C₃-C₆)-Cycloalkyl, Phenyl oder Benzyl, wobei der Phenylkern bis zu dreifach substituiert sein kann mit F, Cl, CF₃, Methyl, Methoxy, NR(9)R(10);
R(9), R(10) unabhängig voneinander H, (C₁-C₄)-Alkyl oder (C₁-C₄)-Perfluoralkyl;
R(F) Wasserstoff;
R(1), R(2), R(3) unabhängig voneinander Wasserstoff, F, Cl, Br, I, CN, -(C=O)- N=C(NH₂)₂, -SO₍₀₋₁₎-(C₁-C₈)-Alkyl , O-(C₀-C₄)-Alkyl-Phenyl, -(C₀-C₄)-Alkyl-Phenyl, wobei der Phenylkern bis zu dreifach substituiert sein kann mit F, Cl, CF₃, Methyl, Methoxy;
L (C₁-C₈)-Alkyl , wobei eine oder mehrere (CH₂)-Gruppen ersetzt sein können durch -CH=CH-, -C≡C-, -O-, -NR(47)-, -NR(48)-, -CO-, -SO₂-;
R(47) Wasserstoff, (C₁-C₄)-Alkyl, R(48)-CO-, Phenyl, (CH₂)-Phenyl;
R(48) Wasserstoff, (C₁-C₄)-Alkyl, Phenyl, (CH₂)-Phenyl, wobei der Phenylkern bis zu dreifach substituiert sein kann mit F, Cl, CF₃, Methyl, Methoxy;
R(41), R(42), R(45) unabhängig voneinander H, -O R(50), -S R(50), NH R(50), -N R(50)₂, -O-(CO)- R(50), -S-(CO)- R(50), -NH-(CO)- R(50), -O-PO-(O R(50))-O R(50), -O-(SO₂)-O R(50), - R(50);
R(50) Wasserstoff, (C₁-C₄)-Alkyl, Phenyl, (CH₂)-Phenyl, wobei der Phenylkern bis zu dreifach substituiert sein kann mit F, Cl, CF₃, Methyl, Methoxy;
K -OR(51), -NH(R51), -N(R51)₂, -HN-CH₂-CH₂-CO₂H, -HN-CH₂-CH₂-SO₃H, -N(CH₃)CH₂CO₂H, -HN-CH(R46)CO₂H, -OKa, wobei Ka ein Kation bedeutet, wie z.B. ein Alkali- oder Erdalkaliion oder ein quartäres Ammoniumion,
R(46) H, C₁-C₄-Alkyl, Benzyl, -CH₂-OH, H₃CSCH₂CH₂-, HO₂CCH₂-, HO₂CCH₂CH₂-;
R(51) H, (C₁-C₄)-Alkyl, Phenyl, (CH₂)-Phenyl, wobei der Phenylrest bis zu dreifach substituiert sein kann mit F, Cl, CF₃, Methyl, Methoxy;
sowie deren pharmazeutisch verträgliche Salze.

3. Verbindungen der Formel I, gemäß Anspruch 1 oder 2, **dadurch gekennzeichnet, daß** darin bedeuten
T1 und T2 unabhängig voneinander 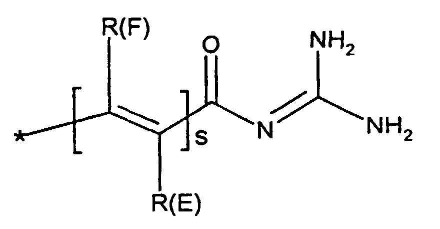 oder Wasserstoff, wobei
T1 und T2 nicht gleichzeitig Wasserstoff sein können;
L-Z 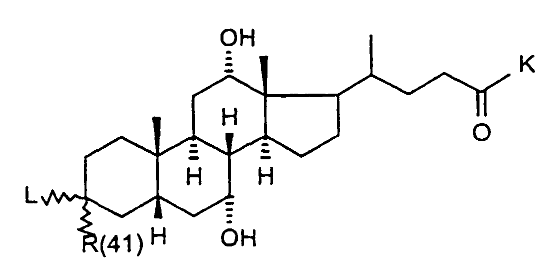
A eine Bindung, -CH₂- , CH₂-X-, -X-;
X -O-, -CO-, -SO₍₀₋₂₎- ;
s Null oder 1;
D Phenylen, welches bis zu zweifach substituiert sein kann mit F, Cl, -CF₃, (C₁-C₄)-Alkyl, Hydroxy, Methoxy, -NH₂, NHCH₃, N(CH₃)₂ , CH₃SO₂-, H₂NO₂S;
R(E) F, (C₁-C₄)-Alkyl, wobei in dem Alkylrest ein, mehrere oder alle Wasserstoffe durch Fluor ersetzt sein können;
R(F) Wasserstoff;
R(1), R(2), R(3) unabhängig voneinander Wasserstoff F, Cl, CN, -SO₂-CH₃, O- (C₀-C₁)-Alkyl-Phenyl, -(C₀-C₁)-Alkyl-Phenyl, wobei der Phenylkern bis zu dreifach substituiert sein kann mit F, Cl, CF₃, Methyl, Methoxy;
L (C₁-C₈)-Alkyl , wobei eine odere mehrere (CH₂)-Gruppen ersetzt sein können durch -CH=CH-, -C≡C-, -O-, -NR(47)-, -NR(48)-, -CO-, -SO₂-;
R(47) Wasserstoff, (C₁-C₄)-Alkyl, R(48)-CO-, Phenyl, (CH₂)-Phenyl;
R(48) Wasserstoff, (C₁-C₄)-Alkyl, Phenyl, (CH₂)-Phenyl, wobei der Phenylkern bis zu dreifach substituiert sein kann mit F, Cl, CF₃, Methyl, Methoxy;
R(41) Wasserstoff, -OH;
K -OR(51), -NH(R51), -N(R51)₂, -HN-CH₂-CO₂H, -HN-CH₂-CH₂-CO₂H, -HN- CH₂-CH₂-SO₃H, -N(CH₃)CH₂CO₂H, -OKa, wobei Ka ein Kation bedeutet, wie z.B. ein Alkali- oder Erdalkaliion oder ein quartäres Ammoniumion;
R(51) H, (C₁-C₄)-Alkyl, Phenyl, (CH₂)-Phenyl, wobei der Phenylrest bis zu dreifach substituiert sein kann mit F, Cl, CF₃, Methyl, Methoxy;
sowie deren pharmazeutisch verträgliche Salze.

4. Verbindungen der Formel I, gemäß einem oder mehreren der Ansprüche 1 bis 3, **dadurch gekennzeichnet, daß** darin bedeuten
T1 und T2 unabhängig voneinander 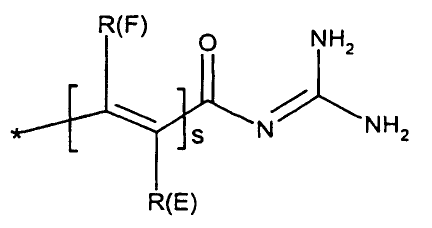 oder Wasserstoff, wobei
T1 und T2 nicht gleichzeitig Wasserstoff sein können;
L-Z 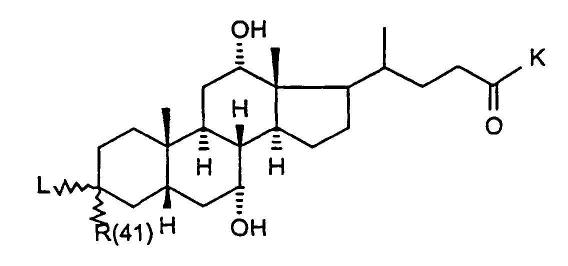
A eine Bindung, -O-;
s Null oder 1;
D Phenylen, welches bis zu zweifach substituiert sein kann mit F, Cl, -CF₃, (C₁-C₄)-Alkyl, Hydroxy, Methoxy, N(CH₃)₂ , CH₃SO₂-, H₂NO₂S;
R(E) (C₁-C₄)-Alkyl;
R(F) Wasserstoff;
R(1), R(2), R(3) unabhängig voneinander Wasserstoff F, Cl, CN, -SO₂-CH₃, O- (C₀-C₁)-Alkyl-Phenyl, -(C₀-C₁)-Alkyl-Phenyl, wobei der Phenylkern bis zu dreifach substituiert sein kann mit F, Cl, CF₃, Methyl, Methoxy;
L (C₁-C₆)-Alkyl, wobei eine odere mehrere (CH₂)-Gruppen ersetzt sein können durch -CH=CH-, -C≡C-, -O-, -NR(47)-, -CO-, -SO₂-;
R(47) Wasserstoff, (C₁-C₄)-Alkyl;
R(41) Wasserstoff, -OH;
K -OH, -HN-CH₂-CO₂H, -HN-CH₂-CH₂-CO₂H, -HN-CH₂-CH₂-SO₃H, -N(CH₃)CH₂CO₂H, -OKa, wobei Ka ein Kation bedeutet, wie z.B. ein Alkali- oder Erdalkaliion oder ein quartäres Ammoniumion;
sowie deren pharmazeutisch verträgliche Salze.

5. Arzneimittel enthaltend eine oder mehrere der Verbindungen gemäß einem oder mehreren der Ansprüche 1 bis 4.

6. Arzneimittel enthaltend eine oder mehrere der Verbindungen gemäß einem oder mehreren der Ansprüche 1 bis 4 und ein oder mehrere lipidsenkende Wirkstoffe.

7. Verfahren zur Herstellung eines Arzneimittels enthaltend eine oder mehrere der Verbindungen gemäß einem oder mehreren der Ansprüche 1 bis 4, **dadurch gekennzeichnet, daß** der Wirkstoff mit einem pharmazeutisch geeigneten Träger vermischt wird und diese Mischung in eine für die Verabreichung geeignete Form gebracht wird.

8. Verwendung der Verbindungen gemäß einem oder mehreren der Ansprüche 1 bis 4 zur Herstellung eines Medikaments zur Prophylaxe oder Behandlung von Gallensteinen.

## Claims

1. A compound of the formula I 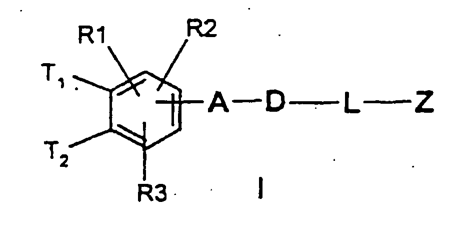 in which:
T1 and T2 independently of one another are 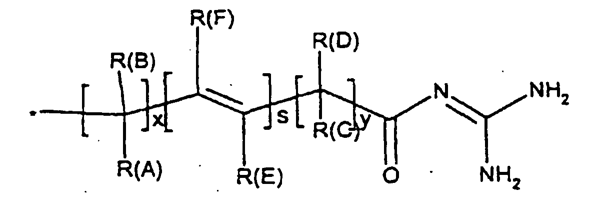 or hydrogen, where
T1 and T2 cannot simultaneously be hydrogen;
Z is 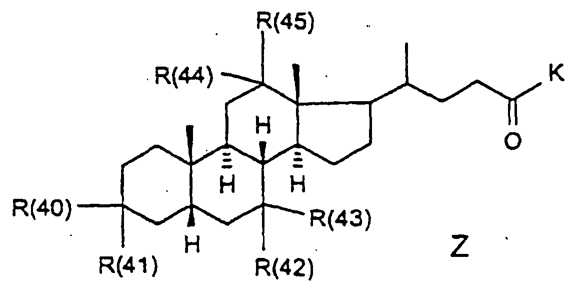
A is a bond, (C₁-C₄)-alkyl, (C₀-C₄)-alkyl-X;
X is -O-, -CO-, -CH[OH]-, -CH[OCH]₃-, -SO₍₀₋₂₎- or -NH-, -N(CH₃)-;
D is phenylene, which is unsubstituted or is substituted by 1 - 3 substituents selected from the group consisting of F, Cl, -CF₃, (C₁-C₄)-alkyl, hydroxyl, methoxy, -NH₂, NHCH₃, N(CH₃)₂, CH₃SO₂- and H₂NO₂S-;
R(A), R(B), R(C), R(D), R(E), R(F) independently of one another are hydrogen, F, Cl,Br, I, CN, OH, OR(6), NR(7)R(8), (C₁-C₈)-alkyl, O-(C₁-C₁₂)-alkyl, (C₃-C₈)-cycloalkyl, it being possible in the alkyl radicals for one, two or more or all hydrogens to be replaced by fluorine;
R(6) is (C₃-C₆)-alkenyl, (C₃-C₈)-cycloalkyl, phenyl or benzyl, where the phenyl nucleus can be up to trisubstituted by F, Cl, CF₃, methyl, methoxy, NR(9)R(10);
R(9), R(10) independently of one another are H, (C₁-C₄)-alkyl or (C₁-C₄)perfluoro-alkyl;
R(7) and R(8) independently of one another are (C₁-C₄)-alkyl, (C₃-C₆)-alkenyl, (C₃-C₈)-cycloalkyl, phenyl or benzyl, where the phenyl nucleus can be up to trisubstituted by F, Cl, CF₃, methyl, methoxy, NR(9)R(10);
or
R(7) and R(8) together form a chain of 4 or 5 methylene groups, of which one CH₂ group can be replaced by oxygen, sulfur, NH, N-CH₃ or N-benzyl;
s is zero or 1,
x is zero, 1 or 2;
y is zero, 1 or 2;
R(1), R(2), R(3) independently of one another are hydrogen, F, Cl, Br, I, CN, -(C=O)-N=C(NH₂)₂, -SO₍₀₋₁₎-(C₁-C₈)-alkyl , O-(C₀-C₄)-alkylphenyl, -(C₀-C₄)alkylphenyl, where the phenyl nucleus can be up to trisubstituted by F, Cl, CF₃, methyl, methoxy, -(C₀-C₄)-alkyl-NR(21)R(22); (C₁-C₈)-alkyl, O-(C₁-C₁₂)-alkyl, (C₃-C₈)-cycloalkyl, it being possible in the alkyl radicals for one, two or more or more hydrogen is to be replaced by fluorine;
R(21), R(22) independently of one another are H, (C₁-C₄)-alkyl;
L is (C₁-C₁₅)-alkyl, it being possible for one or two or more (CH₂) groups to be replaced by -CH=CH-, -C≡C-, -O -NR(47)-, -NR(48)-, -CO-, -SO₂-;
R(47) is hydrogen, (C₁-C₈)-alkyl, R(48)-CO-, phenyl, benzyl;
R(48) is hydrogen, (C₁-C₈)-alkyl, phenyl, (CH₂)-phenyl, where the phenyl nucleus can be up to trisubstituted by F, Cl, CF₃, methyl, methoxy;
R(40) to R(45) independently of one another are H, -O R(50), -S R(50), NH R(50), -N R(50)₂, -O-(CO)- R(50), -S-(CO)- R(50), -NH-(CO)- R(50), -O-PO-(O R(50))-O R(50), -O-(SO₂)-O R(50), -R(50), a bond to L; or
R(40) and R(41), R(42) and R(43), R(44) and R(45) in each case together are the oxygen of a carbonyl group;
where always exactly one of the radicals has the meaning of a bond to L;
R(50) is hydrogen, (C₁-C₄)-alkyl, phenyl, (CH₂)-phenyl, where the phenyl nucleus can be up to trisubstituted by F, Cl, CF₃, methyl, methoxy;
K is -OR(51), -NH(R51), -N(R51)₂, -HN-CH₂-CH₂-CO₂H, -HN- CH₂-CH₂-SO₃H, -N(CH₃)-CH₂CO₂H, -HN-CH(R46)CO₂H, -OCat, where Cat is a cation, such as, for example, an alkali metal or alkaline earth metal ion or a quaternary ammonium ion;
R(46) is hydrogen, C₁-C₄-alkyl, benzyl, -CH₂-OH, H₃CSCH₂CH₂-, HO₂CCH₂-, HO₂CCH₂CH₂-;
R(51) is H, (C₁-C₄)-alkyl, phenyl, (CH₂)-phenyl, where the phenyl radical can be up to trisubstituted by F, Cl, CF₃, methyl, methoxy;
or its pharmaceutically tolerable salts and physiologically functional derivatives.

2. A compound of the formula I according to claim 1, **characterized in that**
T1 and T2 independently of one another are 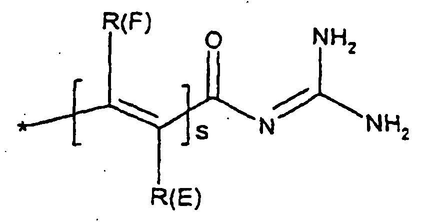 or hydrogen, where
T1 and T2 cannot simultaneously be hydrogen,
L-Z is 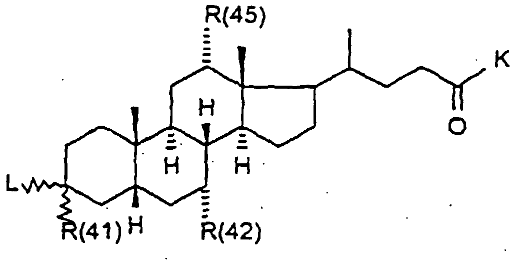
A is a bond -CH₂-, CH₂-X-;
X is -O-, -CO-, -CH[OH]-, -CH[OCH₃]-, -SO₍₀₋₂₎- or -NH-, N(CH₃)-;
s is zero or 1 ;
D is phenylene, which can be up to disubstituted by, F, Cl, -CF₃, (C₁-C₄)-alkyl, hydroxyl, methoxy, -NH₂, NHCH₃, N(CH₃)₂ , CH₂SO₂-, H₂NO₂S-;
R(E) is F, Cl, CN, OR(12), (C₁-C₄)-alkyl, O-(C₁-C₄)-alkyl, (C₃-C₆)-cycloalkyl, it being possible in the alkyl radicals for one, two or more or all hydrogens to be replaced by fluorine;
R(6) is (C₃-C₆)-alkenyl, (C₃-C₆)-cycloalkyl, phenyl or benzyl, where the phenyl nucleus can be up to trisubstituted by F, Cl, CF₃, methyl, methoxy, NR(9)R(10);
R(9), R(10) independently of one another are H, (C₁-C₄)-alkyl or (C₁-C₄)perfluoroalkyl;
R(F) is hydrogen;
R(1), R(2), R(3) independently of one another are hydrogen, F, Cl, Br, I, CN, (C=O)N=C(NH₂)₂, SO₍₀₋₁₎(C₁-C₈)-alkyl , O-(C₀-C₄)alkylphenyl, -(C₀-C₄)-alkylphenyl, where the phenyl nucleus can be up to trisubstituted by F, Cl, CF₃, methyl, methoxy;
L is (C₁-C₈)-alkyl, where one or more (CH₂) groups can be replaced by -CH=CH-, -C≡C-, -O-, -NR(47)-, -NR(48)-, -CO-, -SO₂ -;
R(47) is hydrogen, (C₁-C₄)-alkyl, R(48)-CO-, phenyl, (CH₂)-phenyl;
R(48) is hydrogen, (C₁-C₄)-alkyl, phenyl, (CH₂)-phenyl, where the phenyl nucleus can be up to trisubstituted by F, Cl, CF₃, methyl, methoxy;
R(41), R(42), R(45) independently of one another are H, -O R(50), -S R(50), NH R(50), -N R(50)₂, -O-(CO)- R(50), -S-(CO)- R(50), -NH-(CO)- R(50), -O-PO-(O R(50))-O R(50), -O-(SO₂)- O R(50), - R(50);
R(50) is hydrogen, (C₁-C₄)-alkyl, phenyl, (CH₂)-phenyl, where the phenyl nucleus can be up to trisubstituted by F, Cl, CF₃, methyl, methoxy;
K is -OR(51), -NH(R51), -N(R51)₂, -HN-CH₂-CH₂-CO₂H, -HN- CH₂-CH₂-SO₃H, -N(CH₃)CH ₂CO₂H, -HN-CH(R46)CO₂H, -OCat, where Cat is a cation, such as, for example, an alkali metal or alkaline earth metal ion or a quaternary ammonium ion,
R(46) is H, C₁-C₄-alkyl, benzyl, -CH₂-OH, H₃CSCH₂CH₂-, HO₂CCH₂-, HO₂CCH₂CH₂;
R(51) is H, (C₁-C₄)-alkyl, phenyl, (CH₂)phenyl, where the phenyl radical can be up to trisubstituted by F, Cl, CF₃, methyl, methoxy;
or its pharmaceutically tolerable salts.

3. A compound of the formula I as claimed in claim 1 or 2, **characterized in that**
T1 and T2 independently of one another are 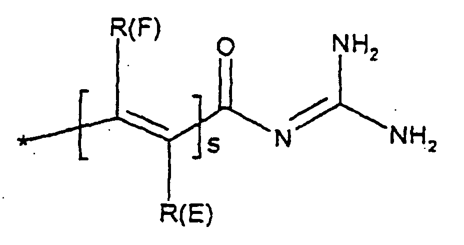 or hydrogen, where
T1 and T2 cannot simultaneously be hydrogen,
L-Z is 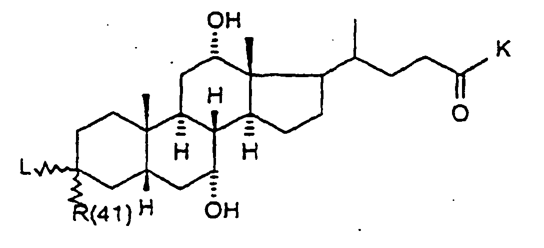
A is a bond, -CH₂-, CH₂-X-, -X-;
X is -O-, -CO-, SO₍₀₋₂₎- ;
s is zero or 1;
D is phenylene, which can be up to disubstituted by F, Cl, -CF₃, (C₁-C₄)-alkyl, hydroxyl, methoxy, -NH₂, NHCH₃, N(CH₃)₂, CH₃SO₂-, H₂NO₂S-;
R(E) is F, (C₁-C₄)-alkyl, it being possible in the alkyl radical for one, two or more or all hydrogens to be replaced by fluorine;
R(F) is hydrogen;
R(1), R(2), R(3) independently of one another are hydrogen, F, Cl, CN, -SO₂-CH₃, O-(C₀-C₁)-alkylphenyl, -(C₀-C₁)-alkylphenyl, where the phenyl nucleus can be up to trisubstituted by F, Cl, CF₃, methyl, methoxy;
L is (C₁-C₈)-alkyl, where one or more (CH₂) groups can be replaced by -CH=CH-, -C≡C-; -O-, -NR(47)-, -NR(48)-, -CO-, -SO₂-;
R(47) is hydrogen, (C₁-C₄)-alkyl, R(48)CO-, phenyl, (CH₂)-phenyl;
R(48) is hydrogen, (C₁-C₄)-alkyl, phenyl, (CH₂)-phenyl, where the phenyl nucleus can be up to trisubstituted by F, Cl, CF₃, methyl, methoxy;
R(41) is hydrogen, -OH;
K is -OR(51), -NH(R51), -N(R51)₂, -HN-CH₂-CO₂H, -HN-CH₂- CH₂-CO₂H, -HN- CH₂-CH₂-SO₃H, ,-N(CH₃)CH₂CO₂H, -OCat, where Cat is a cation, such as,for example, an alkali metal or alkaline earth metal ion or a quaternary ammonium ion;
R(51) is H, (C₁-C₄)-alkyl, phenyl, (CH₂)-phenyl, where the phenyl nucleus can be up to trisubstituted by F, Cl, CF₃, methyl, methoxy;
or its pharmaceutically tolerable salts.

4. A compound of the formula I, as claimed in one or more of claims 1 to 3, **characterized in that**
T1 and T2 independently of one another are 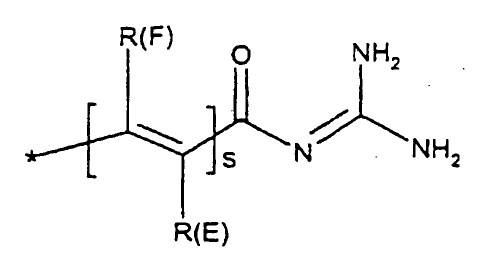 or hydrogen, where
T1 and T2 cannot simultaneously be hydrogen,
L-Z is 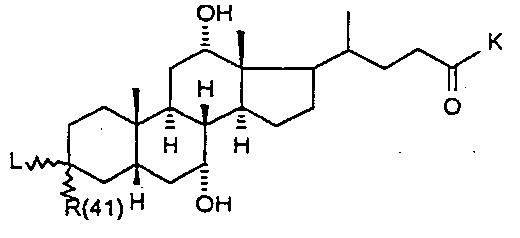
A is a bond, -O-;
s is zero or 1;
D is phenylene, which can be up to disubstituted by F, Cl, -CF₃, (C₁-C₄)-alkyl, hydroxyl, methoxy, N(CH₃)₂ , CH₃SO₂-, H₂NO₂S;
R(E) is (C₁-C₄)-alkyl;
R(F) is hydrogen;
R(1), R(2), R(3) independently of one another are hydrogen, F, Cl, CN, -SO₂-CH₃, O-(C₀-C₁)-alkylphenyl, -(C₀-C₁)-alkylphenyl, where the phenyl nucleus can be up to trisubstituted by F, Cl, CF₃, methyl, methoxy;
L is (C₁-C₆)-alkyl, where one or more (CH₂) groups can be replaced by -CH=CH-, -C≡C-, -O-, -NR(47)-, -CO-, -SO₂-;
R(47) is hydrogen, (C₁-C₄}-alkyl;
R(41) is hydrogen, -OH;
K is -OH, -HN-CH₂-CO₂H, -HN-CH₂-CH₂-CO₂H, -HN-CH₂-CH₂- SO₃H, -N(CH₃)-CH₂CO₂H, -OCat, where Cat is a cation, such as, for example, an alkali metal or alkaline earth metal ion or a quaternary ammonium ion;
or its pharmaceutically tolerable salts.

5. A pharmaceutical comprising one or more of the compounds as claimed in one or more of claims 1 to 4.

6. A pharmaceutical comprising one or more of the compounds as claimed in one or more of claims 1 to 4 and one or more hypolipidemic active compounds.

7. A process for the production of a pharmaceutical comprising one or more of the compounds as claimed in one or more of claims 1 to 4, **characterized in that** the active compound is mixed with a pharmaceutically suitable carrier and this mixture is brought into a form suitable for administration.

8. The use of the compounds as claimed in one or more of claims 1 to 4 for the production of a medicament for the prophylaxis or treatment of gallstones.

## Revendications

1. Composés de formule I 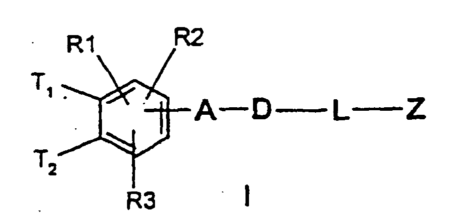 dans laquelle :
T1 et T2 représentent indépendamment l'un de l'autre 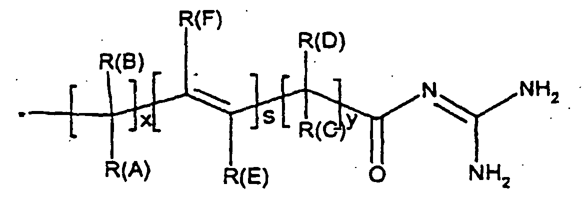 ou un atome d'hydrogène,
T1 et T2 ne pouvant pas être simultanément un atome d'hydrogène ;
Z représente 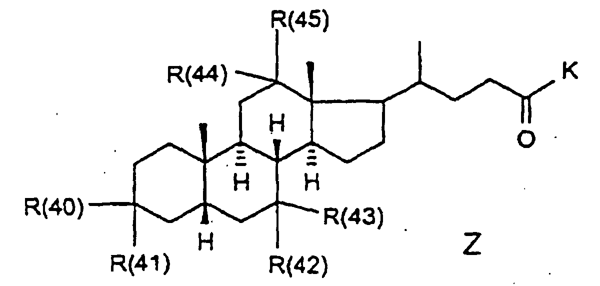
A représente une liaison, un groupe alkyle en C₁-C₄, alkyl(C₀-C₄)-X ;
X représente -O-, -CO-, -CH[OH]-, -CH[OCH₃]-, -SO₍₀₋₂₎- ou -NH-, -N(CH₃)- ;
D représente un groupe phénylène qui n'est pas substitué ou porte de 1 à 3 substituants choisis dans l'ensemble constitué par F, Cl, -CF₃, alkyle en C₁-C₄, hydroxy, méthoxy, -NH₂, NHCH₃, N(CH₃)₂, CH₃SO₂- et H₂NO₂S- ;
R(A), R(B), R(C), R(D), R(E), R(F) représentent, indépendamment les uns des autres, un atome d'hydrogène, F, Cl, Br, I, un groupe CN, OH, OR(6), NR(7)R(8), alkyle en C₁-C₈, O-alkyle(C₁-C₁₂), cycloalkyle en C₃-C₈, un, plusieurs ou tous les atomes d'hydrogène dans les radicaux alkyle pouvant être remplacés par des atomes de fluor ;
R(6) représente un groupe alcényle en C₃-C₆, cycloalkyle en C₃-C₈, phényle ou benzyle, le noyau phényle pouvant être jusqu'à trois fois substitué par F, Cl, CF₃, méthyle, méthoxy, NR(9)R(10) ;
R(9), R(10) représentent, indépendamment l'un de l'autre, H ou un groupe alkyle en C₁-C₄ ou perfluoroalkyle en C₁-C₄,
R(7) et R(8) représentent, indépendamment l'un de l'autre, un groupe alkyle en C₁-C₄, alcényle en C₃-C₆, cycloalkyle en C₃-C₈, phényle ou benzyle, le noyau phényle pouvant être jusqu'à trois fois substitué par F, Cl, CF₃, méthyle, méthoxy, NR(9)R(10) ; ou
R(7) et R(8) forment ensemble une chaîne de 4 ou 5 groupes méthylène, dont un groupe CH₂ peut être remplacé par un atome d'oxygène ou de soufre ou par un groupe NH, N-CH₃ ou N-benzyle ;
s est zéro ou 1 ;
x est zéro, 1 ou 2 ;
y est zéro, 1 ou 2 ;
R(1), R(2), R(3) représentent, indépendamment les uns des autres, un atome d'hydrogène, F, Cl, Br, I, CN, -(C=O)-N=C(NH₂)₂, -SO₍₀₋₁₎-alkyle-(C₁-C₈), O-alkyl(C₀-C₄)-phényle, -alkyl(C₀-C₄)-phényle, le noyau phényle pouvant être jusqu'à trois fois substitué par F, Cl, CF₃, méthyle, méthoxy, -alkyl(C₀-C₄)-NR(21)R(22) ; un radical alkyle en C₁-C₈, O-alkyle(C₁-C₁₂), cycloalkyle en C₃-C₈, un, plusieurs ou tous les atomes d'hydrogène dans les radicaux alkyle pouvant être remplacés par des atomes de fluor ;
R(21), R(22) représentent, indépendamment l'un de l'autre, H ou un groupe alkyle en C₁-C₄ ;
L représente un groupe alkyle en C₁-C₁₅, dans lequel un ou plusieurs groupe CH₂ peuvent être remplacés par -CH=CH-, -C≡C-, -O-, -NR(47)-, -NR(48)-, -CO-, -SO₂- ;
R(47) représente un atome d'hydrogène ou un groupe alkyle en C₁-C₈, R(48)-CO-, phényle, benzyle ;
R(48) représente un atome d'hydrogène, un groupe alkyle en C₁-C₈, phényle, -CH₂-phényle, le noyau phényle pouvant être jusqu'à trois fois substitué par F, Cl, CF₃, méthyle, méthoxy ;
R(40) et R(45) représentent, indépendamment l'un de l'autre, H, -OR(50), -SR(50), NHR(50), -NR(50)₂, -O-(CO)-R(50), -S-(CO)-R(50), -NH- (CO)-R(50), -O-PO-(OR(50))-OR(50), -O-(SO₂)-OR(50), -R(50), une liaison à L ; ou
R(40) et R(41), R(42) et R(43), R(44) et R(45) représentent respectivement ensemble l'atome d'oxygène d'un groupe carbonyle ;
exactement l'un des radicaux ayant toujours la signification d'une liaison à L ;
R(50) représente un atome d'hydrogène ou un groupe alkyle en C₁-C₄, phényle, (CH₂)-phényle, le noyau phényle pouvant être jusqu'à trois fois substitué par F, Cl, CF₃, méthyle, méthoxy ;
K représente -OR(51), -NH(R51), -N(R51)₂, -HN-CH₂-CH₂-CO₂H, -HN-CH₂-CH₂-SO₃H, -N(CH₃)CH₂CO₂H, -HN-CH(R46)CO₂H, -OKa, Ka signifiant un cation, comme par exemple un ion alcalin ou alcalino-terreux ou un ion ammonium quaternaire ;
R(46) représente un atome d'hydrogène ou un groupe alkyle en C₁-C₄, benzyle, -CH₂-OH, H₃CSCH₂CH₂-, HO₂CCH₂-, HO₂CCH₂CH₂- ;
R(51) représente H, un groupe alkyle en C₁-C₄, phényle, (CH₂)-phényle, le radical phényle pouvant être jusqu'à trois fois substitué par F, Cl, CF₃, méthyle, méthoxy ;
ainsi que leurs sels pharmaceutiquement acceptables et dérivés physiologiquement fonctionnels.

2. Composés de formule I selon la revendication 1, **caractérisés en ce que** dans ces composés
T1 et T2 représentent indépendamment l'un de l'autre 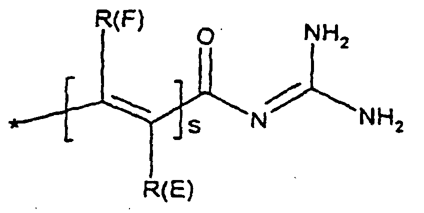 ou un atome d'hydrogène,
T1 et T2 ne pouvant pas être simultanément un atome d'hydrogène ;
L-Z représente 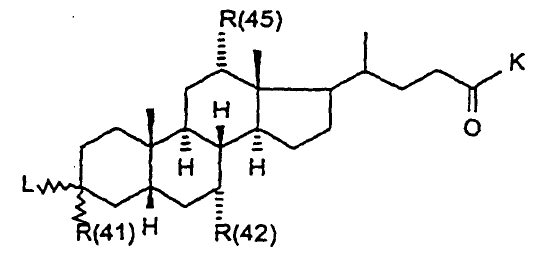
A représente une liaison, -CH₂-, CH₂-X- ;
X représente -O-, -CO-, -CH[OH]-, -CH[OCH₃]-, -SO₍₀₋₂₎- ou -NH-, N(CH₃)- ;
s est zéro ou 1 ;
D représente un groupe phénylène qui peut être jusqu'à deux fois substitué par F, Cl, -CF₃, alkyle en C₁-C₄, hydroxy, méthoxy, -NH₂, NHCH₃, N(CH₃)₂, CH₃SO₂-, H₂NO₂S- ;
R(E) représente F, Cl, CN, OR(12), un radical alkyle en C₁-C₄, O-alkyle(C₁-C₄), cycloalkyle en C₃-C₆, un, plusieurs ou tous les atomes d'hydrogène dans les radicaux alkyle pouvant être remplacés par des atomes de fluor ;.
R(6) représente un groupe alcényle en C₃-C₆, cycloalkyle en C₃-C₆, phényle ou benzyle, le noyau phényle pouvant être jusqu'à trois fois substitué par F, Cl, CF₃, méthyle, méthoxy, NR(9)R(10) ;
R(9), R(10) représentent, indépendamment l'un de l'autre, H ou un groupe alkyle en C₁-C₄ ou perfluoroalkyle en C₁-C₄ ;
R(F) représente un atome d'hydrogène ;
R(1), R(2), R(3) représentent, indépendamment les uns des autres, un atome d'hydrogène, F, Cl, Br, I, CN, -(C=O)-N=C(NH₂)₂, -SO₍₀₋₁₎-alkyle(C₁-C₈), O-alkyl(C₀-C₄)-phényle, -alkyl-(C₀-C₄)-phényle, le noyau phényle pouvant être jusqu'à trois fois substitué par F, Cl, CF₃, méthyle, méthoxy ;
L représente un groupe alkyle en C₁-C₈, dans lequel un ou plusieurs groupes CH₂ peuvent être remplacés par -CH=CH-, -C≡C-, -O-, -NR(47)-, -NR(48)-, -CO-, -SO₂- ;
R(47) représente un atome d'hydrogène, un groupe alkyle en C₁-C₄, R(48)-CO-, phényle, (CH₂)-phényle ;
R(48) représente un atome d'hydrogène ou un groupe alkyle en C₁-C₄, phényle, (CH₂)-phényle, le noyau phényle pouvant être jusqu'à trois fois substitué par F, Cl, CF₃, méthyle, méthoxy ;
R(41), R(42), R(45) représentent, indépendamment les uns des autres, H, -OR(50), -SR(50), NHR(50), -NR(50)₂, -O-(CO)-R(50), -S-(CO)-R(50), -NH-(CO)-R(50), -O-PO-(OR(50))-OR(50), -O-(SO₂)-OR(50), -R(50) ;
R(50) représente un atome d'hydrogène ou un groupe alkyle en C₁-C₄, phényle, (CH₂)-phényle, le noyau phényle pouvant être jusqu'à trois fois substitué par F, Cl, CF₃, méthyle, méthoxy ;
K représente -OR(51), -NH(R51), -N(R51)₂, -HN-CH₂-CH₂-CO₂H, -HN-CH₂-CH₂-SO₃H, -N(CH₃)CH₂CO₂H, -HN-CH(R46)CO₂H, -OKa, Ka signifiant un cation, comme par exemple un ion alcalin ou alcalino-terreux ou un ion ammonium quaternaire ;
R(46) représente H, un groupe alkyle en C₁-C₄, benzyle, -CH₂-OH, H₃CSCH₂CH₂-, HO₂CCH₂-, HO₂CCH₂CH₂- ;
R(51) représente H, un groupe alkyle en C₁-C₄, phényle, (CH₂)-phényle, le radical phényle pouvant être jusqu'à trois fois substitué par F, Cl, CF₃, méthyle, méthoxy ;
ainsi que leurs sels pharmaceutiquement acceptables.

3. Composés de formule I selon la revendication 1 ou 2, **caractérisés en ce que** dans ces composés
T1 et T2 représentent indépendamment l'un de l'autre 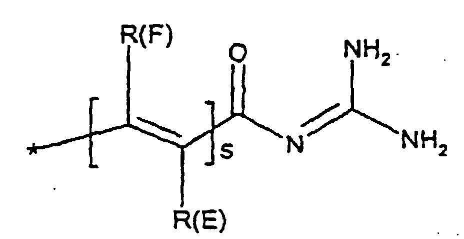 ou un atome d'hydrogène,
T1 et T2 ne pouvant pas être simultanément un atome d'hydrogène ;
L-Z représente 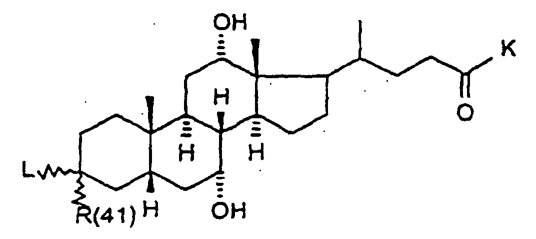
A représente une liaison, -CH₂-, CH₂-X-, -X- ;
X représente -O-, -CO-, -SO₍₀₋₂₎- ;
s est zéro ou 1 ;
D représente un groupe phénylène qui peut être jusqu'à deux fois substitué par F, Cl, -CF₃, alkyle en C₁-C₄, hydroxy, méthoxy, -NH₂, NHCH₃, N(CH₃)₂, CH₃SO₂-, H₂NO₂S- ;
R(E) représente F, un groupe alkyle en C₁-C₄, un, plusieurs ou tous les atomes d'hydrogène dans le radical alkyle pouvant être remplacés par des atomes de fluor ;
R(F) représente un atome d'hydrogène ;
R(1), R(2), R(3) représentent, indépendamment les uns des autres, un atome d'hydrogène, F, Cl, CN, -SO₂-CH₃, O-alkyl(C₀-C₁)-phényle, -alkyl-(C₀-C₁)-phényle, le noyau phényle pouvant être jusqu'à trois fois substitué par F, Cl, CF₃, méthyle, méthoxy ;
L représente un groupe alkyle en C₁-C₈, dans lequel un ou plusieurs groupes CH₂ peuvent être remplacés par -CH=CH-, -C≡C-, -O-, -NR(47)-, -NR(48)-, -CO-, -SO₂- ;
R(47) représente un atome d'hydrogène, un groupe alkyle en C₁-C₄, R(48)-CO-, phényle, (CH₂) -phényle ;
R(48) représente un atome d'hydrogène, un groupe alkyle en C₁-C₄, phényle, (CH₂)-phényle, le noyau phényle pouvant être jusqu'à trois fois substitué par F, Cl, CF₃, méthyle, méthoxy ;
R(41) représente un atome d'hydrogène, -OH ;
K représente -OR(51), -NH(R51), -N(R51)₂, -HN-CH₂-CO₂H, -HN-CH₂-CH₂-CO₂H, -HN-CH₂-CH₂-SO₃H, -N(CH₃)CH₂CO₂H, -OKa, Ka signifiant un cation, comme par exemple un ion alcalin ou alcalino-terreux ou un ion ammonium quaternaire ;
R(51) représente H, un groupe alkyle en C₁-C₄, phényle, (CH₂)-phényle, le radical phényle pouvant être jusqu'à trois fois substitué par F, Cl, CF₃, méthyle, méthoxy ;
ainsi que leurs sels pharmaceutiquement acceptables.

4. Composés de formule I selon une ou plusieurs des revendications 1 à 3, **caractérisés en ce que** dans ces composés
T1 et T2 représentent indépendamment l'un de l'autre 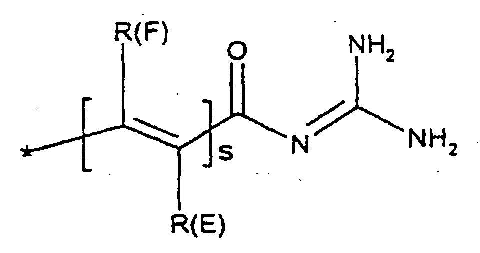 ou un atome d'hydrogène,
T1 et T2 ne pouvant pas être simultanément un atome d'hydrogène ;
L-Z représente 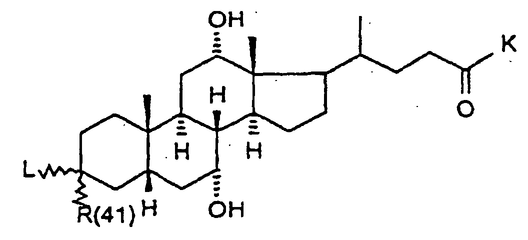
A représente une liaison, -O- ;
s est zéro ou 1 ;
D représente un groupe phénylène qui peut être jusqu'à deux fois substitué par F, Cl, -CF₃, alkyle en C₁-C₄, hydroxy, méthoxy, NH(CH₃)₂, CH₃SO₂-, H₂NO₂S- ;
R(E) représente un groupe alkyle en C₁-C₄ ;
R(F) représente un atome d'hydrogène ;
R(1), R(2), R(3) représentent, indépendamment les uns des autres, un atome d'hydrogène, F, Cl, CN, -SO₂-CH₃, O-alkyl(C₀-C₁)-phényle, -alkyl-(C₀-C₁)-phényle, le noyau phényle pouvant être jusqu'à trois fois substitué par F, Cl, CF₃, méthyle, méthoxy ;
L représente un groupe alkyle en C₁-C₆, dans lequel un ou plusieurs groupes CH₂ peuvent être remplacés par -CH=CH-, -C≡C-, -O-, -NR(47)-, -CO-, -SO₂- ;
R(47) représente un atome d'hydrogène, un groupe alkyle en C₁-C₄ ;
R(41) représente un atome d'hydrogène, -OH ;
K représente -OH, -HN-CH₂-CO₂H, -HN-CH₂-CH₂-CO₂H, -HN-CH₂-CH₂-SO₃H, -N(CH₃)CH₂CO₂H, -OKa, Ka signifiant un cation, comme par exemple un ion alcalin ou alcalino-terreux ou un ion ammonium quaternaire ;
ainsi que leurs sels pharmaceutiquement acceptables.

5. Médicament contenant un ou plusieurs des composés selon une ou plusieurs des revendications 1 à 4.

6. Médicament contenant un ou plusieurs des composés selon une ou plusieurs des revendications 1 à 4 et une ou plusieurs substances actives hypolipidémiantes.

7. Procédé pour la fabrication d'un médicament contenant un ou plusieurs des composés selon une ou plusieurs des revendications 1 à 4, **caractérisé en ce que** la substance active est mélangée avec un véhicule pharmaceutiquement approprié, et on met ce mélange sous une forme appropriée à l'administration.

8. Utilisation des composés selon une ou plusieurs des revendications 1 à 4, pour la fabrication d'un médicament destiné à la prophylaxie ou au traitement de calculs biliaires.
